# EUROPEAN PATENT SPECIFICATION

(11) **EP 3 440 518 B1**
(45) Date of publication and mention of the grant of the patent: **05.11.2025**
(21) Application number: 17714807.9
(22) Date of filing: 04.04.2017
(51) Int. Cl.: G05B 17/02, G05B 23/02

(54) **REAL TIME MONITORING OF PRODUCT PURIFICATION**
ECHTZEITÜBERWACHUNG VON PRODUKTREINIGUNG
SURVEILLANCE EN TEMPS RÉEL DE LA PURIFICATION DE PRODUITS

(30) Priority: 04.04.2016 EP 16163713
(43) Date of publication of application: 13.02.2019
(73) Proprietor: Boehringer Ingelheim RCV GmbH & Co KG, 1121 Wien (AT); SANDOZ GMBH, 6250 Kundl (AT)
(72) Inventor: JUNGBAUER, Alois, 1190 Wien (AT); DÜRAUER, Astrid, 1190 Wien (AT); WALCH, Nicole, 1190 Wien (AT); SAUER, Dominik, 1190 Wien (AT); SCHARL-HIRSCH, Theresa, 1190 Wien (AT); MELCHER, Michael, 1190 Wien (AT); LEISCH, Friedrich, 1190 Wien (AT)
(74) Representative: Schiweck Weinzierl Koch Patentanwälte Partnerschaft mbB
(86) International application number: PCT/EP2017/057986
(87) International publication number: WO 2017/174580

(56) References cited:
- US-A1- 2008 109 090
- US-A1- 2009 312 851
- US-A1- 2010 191 361

## Description

### Field of the Invention

The present invention relates to the field of monitoring and controlling a purification and/or concentration process of a biological product and devices for use in these methods. Specifically, the invention relates to a method for online monitoring and controlling of protein concentration, purity, and potency and for parametric or real time release.

### Background Art

During downstream processing, biological products are purified and concentrated in accordance to their applications and needs. A sequence of different purification process steps are usually carried out to achieve desired purity, concentration and potency of the product, which is measured off-line. Overexpression in microbial or mammalian cells is particularly used for recombinant production of biological products, such as biopharmaceuticals. Such compounds may include, for example, proteins (e.g. antibodies and fragments thereof), nucleic acids, carbohydrates, lipids, organic small molecules, non-organic small molecules, viruses, liposomes, and hybrids or variant forms of any such compounds.

Downstream processing of such recombinantly produced compounds from cell culture requires purification of the compound to a state where impurities, for example, but not limited to, host cell proteins (HCP), host cell DNA, viruses, cellular debris, lipids, cell culture media components and product related impurities such as fragments, aggregates and free light chains, are reduced to levels defined in the product specification. This requirement is especially mandatory for therapeutic products.

A purification process for a soluble secreted biological product, such as a biopharmaceutical, comprises cell removal by centrifugation, flocculation, microfiltration or filtration or combinations of these listed processes. Purification is usually achieved by a combination of chromatography and membrane filtration processes. In case of a mammalian cell expression system two orthogonal dedicated virus inactivation processes must also be included. Some purification processes also use precipitation and crystallization steps.

If the biological product is not secreted, the cells must be disrupted and the homogenate must be clarified for further purification.

For biological products deposited as inclusion bodies, a refolding/oxidation process must be performed in addition.

Generally, in manufacturing of a biological product such as a biopharmaceutical, ion exchange chromatography, hydrophobic interaction chromatography, affinity chromatography and mixed mode chromatography are used to purify and/or concentrate the biological product. Due to low productivity, size exclusion chromatography is used to a lesser extent. Chromatography is either performed in a bind-elute or flow through mode. For high resolution gradients of mobile phase, modulators such as salt or pH are applied. Besides the operation parameters and quality of the chromatography column, the composition of the feed stock is the major parameter influencing the performance of the separation by a chromatography column.

The composition of the feed stock is determined by the media components, the expression host, and the fermentation procedure, which includes the time point of harvest. A fermentation broth consists of water (> 80%), unused and digested media components, compounds secreted by the cell, all cell components from lysed cells, product and product variants, antifoam oil, cells and cell debris. Compared to upstream processing, after chromatography and/or filtration processes, the solutions containing the biological product are almost transparent, although slightly turbid solutions may be present especially during the capture step.

Biophysical characteristics of the various components present in a fermentation broth may be distinct from or very similar to the product itself, so that several measurements on the column effluent must be used which allow discriminating between product and impurities, to quantify the product and impurity and to quantify the potency of the product. A single analytical method is not capable to manage this measurement and discrimination at the present time.

A biological product is characterized by its physicochemical properties, biological activity (potency), immunochemical properties, purity, impurities, and contaminants according to the guidelines of International committee of harmonization (ICH Guidelines) of the European Medicines Agency (EMEA). For biological products, the substance can include several molecular entities or variants. Therefore, an absolute purity and a relative purity (units of biological activity per mg of product) have been defined and test procedures as well as acceptance criteria were standardized by the ICH of the EMEA (ICH Q6B published September 1999). According to the ICH Guidelines, the purity of the biological substance and biological product is assessed by a combination of analytical procedures, which are measured after the purification process has been conducted. The specific activity of a biological product, also called potency of the product, is highly process or product dependent.

Contaminants in a biological product according to ICH Guidelines include all adventitiously introduced materials not intended to be part of the manufacturing process, such as chemical and biochemical materials e.g., microbial proteases, and/or microbial species. Contaminants should be strictly avoided and/or suitably controlled with appropriate in-process acceptance criteria or action limits for drug substance or drug product specifications. For viral, mycoplasma, and prion contamination the concept of "action limit" is not valid. In such a case the starting material should be preferably free of agent, spiking experiments to demonstrate clearance must be conducted, and the final product must be controlled. These action limits are defined in the ICH Harmonized Tripartite Guidelines: Quality of Biotechnological/Biological Products: Viral Safety Evaluation of Biotechnology Derived Products Derived from Cell Lines of Human or Animal Origin (Q5A); Quality of Biotechnological/Biological Products: Derivation and Characterization of Cell Substrates Used for Production of Biotechnological/Biological Products (Q5D).

In the ICH Guidelines, process related impurities and product related impurities are differentiated. Critical impurities in a biological product are defined as compounds which may directly harm the patients, such as product aggregates, degradation products of toxin conjugates, toll-like receptor activators, growth factors or cytokines released from host cells. Product related impurities may be more difficult to discriminate than process related impurities, due to high similarities of biophysical characteristics to the biological product. Typical product related impurities are molecular variants arising during manufacture and/or storage, which do not have properties comparable to those of the desired product with respect to activity, efficacy, and safety. Examples of product related impurities are precursors, certain degradation products, aberrant glycoforms, or aggregates. Thus a product related impurity can be a critical one or a non-critical one. For example, aberrant glycoforms represent a critical product related impurity [1].

Measurement of all these properties (potency, quantity/concentration, purity/level of impurities) of the biological product according to the ICH Guidelines is currently done with off-line methods, where a sample of the final product is taken to ensure the quality of the product conforms to the standards.

In general, decisions in a production process during downstream processing and final releasing of a biological product such as a biopharmaceutical are based on off-line analyses. While often on-line monitors such as UV, pH, conductivity, pressure are used for monitoring the stability of a process independent of the intended product. Thus for example, the chromatographic runs in the Examples were performed on an Äkta Pure 25 system (GE Healthcare, Sweden), which is equipped with standard sensors for UV-VIS, conductivity, pH and pressure to ensure that the device is functioning properly. However, for monitoring the concentration, purity or potency of the intended product, samples from fractions of column effluents, membrane retentates or refolding solutions must be drawn and then analyses on quantity, purity and potency are made offline. In some conventional online monitoring systems, product concentration and impurities seen by UV- and/or IR spectroscopy are monitored together with pH and/or conductivity. Such methods are not suited for real time release, because not all impurities are quantified and the biological activity, often referred to as potency, is not determined by these conventional on-line monitoring systems [10]. Online monitoring data and off-line analysis are then generally combined to decide if the intermediate of the biological product is within specification limits and suited for further processing or for release in the last stage of bioprocessing. Document US2009/312851 A1 discloses a method to monitor and control a bioprocess using multivariate statistical analysis.

As mentioned above, in the prior art, spectroscopic methods such as mid-range FTIR have been used to monitor composition of biological fluids or effluents of chromatographic columns, but a real time measurement of purity, potency and/or quantity has never be taken into consideration [2]. This is judged as not possible. It has been also suggested to use spectroscopy in atline mode [3,4]. In atline monitoring, the sample is drawn and further manipulated and the result is not obtained in real time. The result is obtained with a certain delay. In off-line analysis the sample is removed from the process stream and analyzed later manually, while the process continues, so that there is a time lag between sample capture and analysis. By using these methods, individual components can be quantified [5] or a certain impurity can be monitored [6,7], but a holistic picture with respect to the purity, potency and/or quantity of the biological product at a given time point during or right after the process is not obtained [2]. Indeed, what has been described as online monitoring is often atline, because a sample is removed and there is a time lag between the time of sample extraction and the analysis of the sample. In online and inline methods, the time in which information about process or material properties is obtained is shorter than the time in which these properties change [8]. Often methods have been referred to as online although they are atline, because the sample is further manipulated [9]. For a slow process, atline or fast offline methods may be still suited for process control or intervention in a running process. However, for fast processes only on- and inline methods are suited. For biological products and specifically biopharmaceutical manufacturing processes, inline or *in situ* monitoring must be non-invasive, because process stream properties must not be altered by the monitoring procedure. The change of the process stream properties during processing dictates the speed of the monitoring and the analytical methods which are suited to monitoring the process. For the purification of biopharmaceuticals, often very fast analytical methods are required in order to intervene. Properties of the process stream may change within the range of several seconds.
For this reason, it is desirable to provide an improved method for online monitoring and/or for controlling the downstream processing of a biological product, such as a recombinantly produced biopharmaceutical.

### Summary of invention

The objective is addressed by the subject matter as claimed.

Methods and devices for monitoring and optionally controlling downstream process parameters are provided.

Embodiments of the invention include applying online sensors and preferably non-invasive *in situ* and/or inline sensors in operation units to obtain process data and implementing multivariate statistical analysis for monitoring and control of the purification and/or concentration process or parts thereof.

Specifically, the present invention relates to a computer based method for monitoring the purification and/or concentration process of a biological product as defined in the claims.

In certain embodiments of the methods of the invention, at least one of the online sensors is selected from the group consisting of multi-angle light scattering sensors (MALS), UV-VIS absorption sensors, fluorescence sensors, attenuated total reflection-fourier transform infrared spectroscopy sensors (ATR-FTIR), refractive index (RI) sensors, pH sensors, temperature sensors, conductivity sensors, pressure sensors, small angle x-ray scattering (SAXS) sensors and redox sensors. In one embodiment, at least one of the online sensors is selected from the group consisting of ATR-FTIR, MALS, RI and fluorescence sensors. In certain embodiments, at least one of the online sensors is a non-invasive *in situ* sensor, preferably selected from the group consisting of ATR-FTIR, SAXS, temperature, pH, conductivity and redox sensors.

In the method of the invention, the operation unit can comprise at least three, at least four, at least five, at least 6, at least 7, at least 8, at least 9, at least 10 or more independent online sensors. In one embodiment, the operation unit comprises at least one ATR-FTIR sensor, one MALS sensor, one RI sensor and one fluorescence sensor and optionally at least two temperature sensors, at least one conductivity sensor, at least one pH sensor and at least one pressure sensor. Preferred sensors are non-invasive *in-situ* sensors selected from the group consisting of SAXS sensors, ATR-FTIR sensors, temperature sensors, conductivity sensors, pH sensors and pressure sensors.

The operation unit used in the methods of the invention may comprise at least one chromatography unit and/or filtration unit. The chromatography unit may be selected from the group consisting of ion exchange chromatography, affinity chromatography, size exclusion chromatography
, reversed phase chromatography, hydrophobic interaction chromatography, multi-modal resin chromatography, operated in isocratic, linear, segmented and/ or step gradient elution in bind/elute or flow through mode and the filtration unit is selected from the group consisting of ultrafiltration, microfiltration, nanofiltration, depth filtration, operated in tangential flow filtration, dead end filtration, filtration through absolute pore size membranes.

The methods of the invention allow the purification and/or concentration process to be regulated with regard but not limited to any one or a combination of peak collection, correct collection of the biological product after refolding, filtration or precipitation, product quality, economy, environmental aspects, energy consumption, and process equipment maintenance.

The methods of the invention may be used for the purification and/or concentration of any biological product, such as a biopharmaceutical e.g. a nucleic acid molecule or a heterologous protein. Preferred proteins are therapeutic proteins, enzymes and peptides, protein antibiotics, fusion proteins, carbohydrate - protein conjugates, structural proteins, regulatory proteins, vaccines and vaccine like proteins or particles, process enzymes, growth factors, hormones and cytokines or antibodies.

In a preferred embodiment of the method of the invention, the concentration, purity and potency of the biological product is predicted in step c) and monitored in step e) of the method.

The invention also relates to a method for producing a biological product as defined in the claims.

The invention also provides a device comprising an operation unit for purification and/or concentration of a biological product as defined in the claims.

Preferred non-invasive *in-situ* sensors to be used in the devices of the invention can be selected from the group consisting of temperature sensors, SAXS sensors, pH sensors, conductivity sensors, ATR-FTIR sensors and redox sensors. In a preferred embodiment, the device comprises at least one further online sensor selected from the group consisting of multi-angle light scattering sensors (MALS), UV-VIS absorption sensors, fluorescence sensors, infrared absorption sensors (IR), attenuated total reflection-fourier transform infrared spectroscopy sensors (ATR-FTIR), light refractive index (RI) sensors , pH sensors, temperature sensors, conductivity sensors, pressure sensors, SAXS sensors and redox sensors.
Further embodiments of the invention, features and advantages, as well as structure and operation of various embodiments are described in detail below.

### Figure legend

**Fig. 1****:** Schematic overview of tools and principles of a real-time monitored downstream process.
**Fig. 2****:** Flow diagram of a chromatography system equipped with UV-VIS detector, conductivity probe, ATR-FTIR probe, fluorescence, light scattering and refractive index detector as well as pH probe connected in series. Chromatography system consists of modules (system/sample pumps, valves, fraction collector columns, sensors (pH, conductivity, UV)) and is additionally equipped with above mentioned detectors.

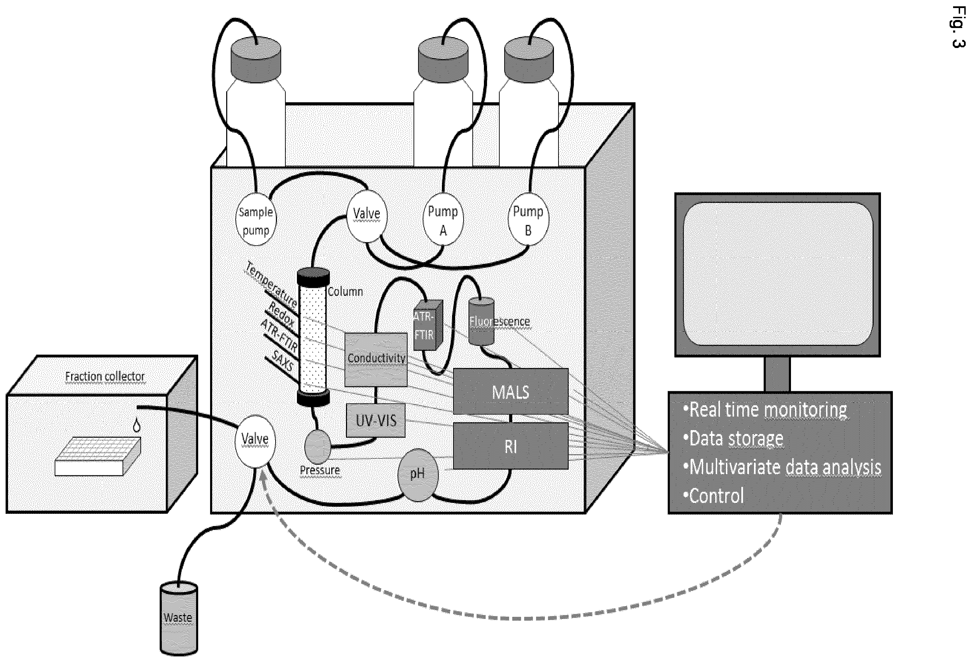

**Fig. 3****:** Schematic drawing of online monitoring device comprising of a chromatography column with pumps, valves, fraction collector and sensors as well as a computer for data storage, multivariate data analysis, real time monitoring and control. The sensors are a pressure sensor, a UV-VIS sensor, a conductivity sensor, and a pH sensor, which are standard detectors of the purification unit (chromatography column), as well as an ATR-FTIR sensor, a fluorescence sensor, a MALS sensor and a RI sensor for inline measurements and a temperature sensor, a redox sensor, an ATR-FTIR sensor, and a SAXS sensor installed for non-invasive *in* situ measurements. The dashed arrow indicates possible real-time control of the process based on the statistical model.
**Fig. 4****:** Schematic drawing of chromatography column equipped with non-invasive *in situ sensor*s: two temperature sensors, a SAXS sensor, an ATR-FTIR sensor, and a redox sensor exemplarily. A) Exterior and B) interior view.
**Fig. 5****:** Sequence of operations of real-time monitored downstream process. In this Figure, offline data are also used as a further control before release.
**Fig. 6****:** Chromatogram of an antibody capture by application of a mAb Select SuRe^{®} column (CV: 21.9 ml). 10 CV of filtered supernatant were loaded. Elution was performed by a step gradient at pH 3.5 over 10 CV.
**Fig. 7****:** Chromatogram FGF-2 purification by application of a CM Sepharose^{®} Fast Flow column (CV: 12.3 ml). 10 CV of clarified supernatant were loaded. Elution of FGF-2 performed by 0 - 1M NaCl gradient over 5 CV.
**Fig. 8****:** Schematic illustration of an ultrafiltration/diafiltration (UF/DF) process with integrated online monitors.
**Fig. 9****:** Chromatogram of an FGF-2 purification (chromatography) by application of a CM Sepharose^{®} Fast Flow column (CV: 12.3 ml) monitored by UV₂₈₀ and static light scattering sensors (SLS) over time / volume of eluates.
**Fig. 10****:** Elution peak of the chromatogram of an FGF-2 purification of Fig. 9 monitored by UV₂₈₀ and static light scattering sensors (SLS) in correlation with potency, purity and quantity measured offline by eluate sampling.
**Fig. 11****:** ATR-FTIR spectra over time / volume of eluates of an FGF-2 purification run (chromatography): 3D Chromatogram shows changes of the ATR-FTIR spectra over time / volume of eluates.
**Fig. 12****:** Zoom into the 3D chromatogram of the FGF-2 purification run of Fig. 11: 3D Chromatogram shows changes of the ATR-FTIR spectra over time / volume of eluates.
**Fig. 13****:** ATR-FTIR spectral change of the intensity of two wavelengths (1528 cm⁻¹ and 1622cm⁻¹) over the course of a purification run (chromatography). The signals lie within the amide bands of an MIR spectrum.
**Fig. 14****:** Differential refractive index (dRI) signal of an FGF-2 purification run (chromatography). Process phases of the chromatographic process can be clearly distinguished. In the elution phase the contribution of the eluting protein to the RI signal can be seen.

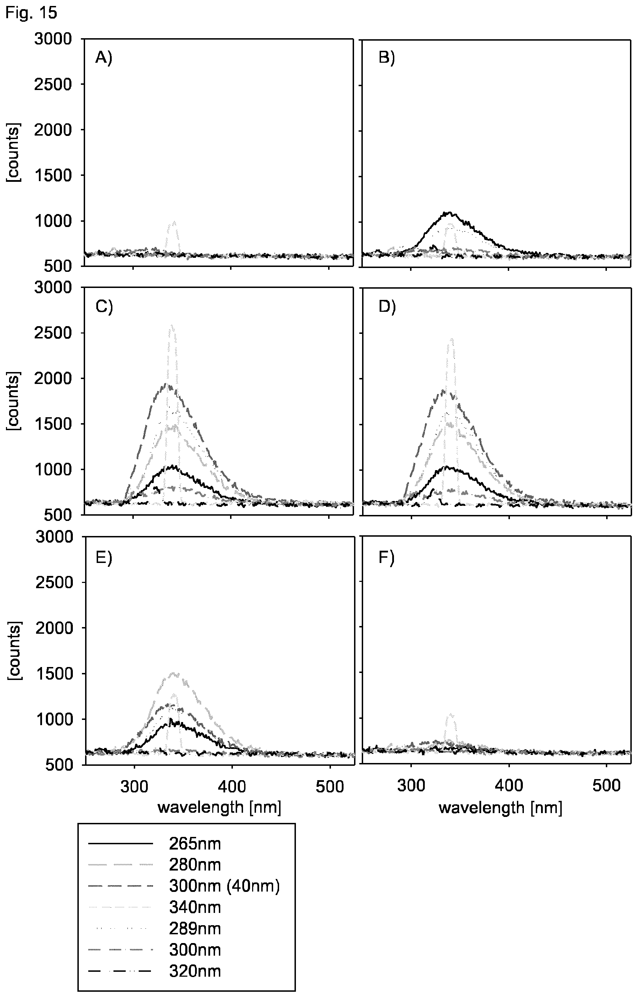

**Fig. 15****:** Fluorescence spectra of the elution peak of an FGF-2 purification (chromatography) process over time. 7 different excitation wavelengths were used, emission spectra from 236 -795 nm were recorded. Fluorescence spectra A: 0 % of elution buffer (100 mM Na-phosphate, 1 M NaCl, pH 7.0). No fluorescence signal detected as no proteins are eluting. Fluorescence spectra B: 25 % of elution buffer (100 mM Na-phosphate, 1 M NaCl, pH 7.0). Increase of fluorescence signal is detected as target protein starts to elute. Fluorescence spectra C: 35 % of elution buffer (100 mM Na-phosphate, 1 M NaCl, pH 7.0). Peak maximum of fluorescence signal is detected. Highest concentration of target protein in eluate. Fluorescence spectra D: 45 % of elution buffer (100 mM Na-phosphate, 1 M NaCl, pH 7.0). Fluorescence signals start slightly to decrease. Target protein concentration in eluate becomes lower. Fluorescence spectra E: 55 % of elution buffer (100 mM Na-phosphate, 1 M NaCl, pH 7.0). Fluorescence signals show a shift in signal intensity and peak maxima compared to spectra A-D as target protein together with host cell protein is eluting. Fluorescence spectra F: 70 % of elution buffer (100 mM Na-phosphate, 1 M NaCl, pH 7.0). Fluorescence signals caused by eluting host cell proteins. Fluorescence signals maxima show shift to higher wavelength compared to fluorescence spectra of pure target protein.
**Fig. 16****:** Breakthrough curve calculated using multivariate analysis for loading an FGF-2 supernatant onto a 10 ml Heparin-SFF column. Loading of column stopped when breakthrough (C/C₀) > 10 %. C is the concentration of target protein FGF-2 in the flow through and C₀ the concentration of FGF-2 in the feed load. 10 % of FGF-2 are not retained by the resin and are present in the flow through.
**Fig. 17****:** Performance of a prediction model for the quantity of the biological product. Cross-validated model predictions on a time grid of 1 second (curve) and their fraction-wise averages (horizontal line) can be compared to the offline measured quantity values (shaded bars) resulting in an error of 0.25 mg/ml.
**Fig. 18****:** Prediction performance of a host cell protein (HCP, in ng/ml) prediction model visualized for run 3 with an error of RMSE = 44 ng/ml. For a single fraction (minutes 5-6) no offline data are available. Cross-validated model predictions on a time grid of 1 second (curve) and their fraction-wise averages (horizontal line) can be compared to the offline measured HCP values (shaded bars) resulting in an error of 44 ng/ml.
**Fig. 19****:** Concentration of host cell protein (HCP) relative to the protein quantity (in units ppm = ng HCP per mg protein) as determined by the ratio of the modeled HCP and quantity curves. Fractions with (near) zero or negative quantity estimates must be omitted. Cross-validated model predictions of both models for protein quantity and HCP combined on a time grid of 1 second (curve) and their fraction-wise averages (horizontal line) can be compared to the offline measured values (shaded bars).
**Fig. 20****:** Results of the application of a dsDNA (doublestranded DNA) model yielding a prediction error of about 41 ng/ml for run 3. Cross-validated model predictions on a time grid of 1 second (curve) and their fraction-wise averages (horizontal line) can be compared to the offline measured dsDNA values (shaded bars).
**Fig. 21****:** Performance of the dsDNA prediction model in units of ng dsDNA per mg protein (equivalent to ppm) for run 3 relative to the protein quantity (in units ppm = ng dsDNA per mg protein) as determined by the ratio of the modeled dsDNA and quantity curves. Cross-validated model predictions on a time grid of 1 second (curve) and their fraction-wise averages (horizontal line) can be compared to the offline values (shaded bars).
**Fig. 22****:** Results of the monomer prediction model depicted for run 3 (RMSE = 0.08, average RMSE = 0.055). Cross-validated model predictions on a time grid of 1 second (curve) and their fraction-wise averages (horizontal line) can be compared to the offline measured monomer values (shaded bars).
**Fig. 23****:** High molecular weight (HMW) impurities as predicted by a statistical model for run 3. The performance values are identical to those of the monomer fraction, as these two targets always sum up to one. Cross-validated model predictions on a time grid of 1 second (curve) and their fraction-wise averages (horizontal line) can be compared to the offline measured HMW values (shaded bars)
**Fig. 24****:** Performance of a potency prediction model using several online signals (UV-VIS, Conductivity, Pressure, pH, MALS, RI, Fluorescence and ATR-FTIR). Cross-validated model predictions on a time grid of 1 second (curve) and their fraction-wise averages (horizontal line) can be compared to the offline measured potency (KD) values (shaded bars).

### Definitions

Unless otherwise stated, the following terms used in this document, including the description and claims, have the definitions given below. Those skilled in the art will recognize, or be able to ascertain, using not more than routine experimentation, many equivalents to the specific embodiments of the invention described herein. Such equivalents are intended to be encompassed by the present invention.

The term "biological product" as used herein refers to a product resulting from a biological process, such as microbial fermentation broth or cell culture to be further processed using the methods of the invention. A biological product may be a biopharmaceutical. Examples of biological products are: a nucleic acid molecule or heterologous protein, preferably selected from therapeutic proteins, enzymes and peptides, protein antibiotics, fusion proteins, carbohydrate - protein conjugates, structural proteins, regulatory proteins, vaccines and vaccine like proteins or particles, process enzymes, growth factors, hormones and cytokines, antibodies or a metabolite of said biological product. The biological product is existent in the different stages / steps of a downstream process (purification and/or concentration process) in fluids or suspensions and the like having different matrices, depending on whether it is an early or a late process step. The matrix comprises process, cell and product related impurities, like host cell proteins (HCP), genomic DNA, RNA, high molecular weight (HMW) impurities, endotoxins, lipids, cell debris, precipitated proteins, product variants and the like. The biological fluid further is existent in the different stages / steps of a process in different concentrations and may have different potency.

The term "concentration" with respect to the biological product is used interchangeably with the term "quantity". The concentration is measured in mg/ml.

The term "purity" as used herein refers to the amount product present in the biological product related to present impurities. Purity may be expressed as % desired variant of the biological product (e.g.: supercoiled form of plasmid DNA or protein with the amino acid sequence and the N-terminus of the naturally occurring protein) of the sum of all variants of the biological product) or % monomeric form of the sum of all forms of the biological product. Purity may also be expressed as mg or µg or ng of an impurity per milliliter of the fluid or suspension comprising the biological product (mg or µg or ng / mL) or as mg or µg or ng per milligram of the biological product in the fluid or suspension (mg or µg or ng / mL or also expressed as ppm or ppb (parts per million, parts per billion). An absolute purity and a relative purity (units of biological activity per mg of product) have been defined and test procedures as well as acceptance criteria were standardized by the ICH of the EMEA (ICH Q6B published September 1999). As mentioned above, in the ICH Guidelines, process related impurities and product related impurities are differentiated. In the context of the Examples, product purity is determined as concentration of the monomeric product and related to host cell protein concentration (HCP) or DNA concentration and high molecular weight (HMW) impurities. Impurities comprise host / cell related impurities (such as but not limited to host cell proteins (HCP), genomic DNA, RNA, high molecular weight (HMW) impurities, endotoxins, lipids, cell debris, precipitated proteins and the like), process related impurities (such as but not limited to antifoam, antibiotics, Tween, detergents, cyclodextrins and any other compounds added to the process and the like) and product related impurities (such as product variants, product aggregates, truncated forms of the product and the like).

The term "potency" refers to the biological activity of the biological product and is measured in as the equilibrium dissociation constant KD value of the binding of the specific biological product to a receptor or another molecule triggering the biological activity. The equilibrium binding constant can be obtained by measuring the binding kinetics in a biosensors, receptor binding assay with radio labeled fluorescent labeled ligands, or by calorimetric assay such as isothermal titration calorimetry. Another way to obtain potency is to measure the 50% of the effective concentration (EC₅₀) of a biological product in a cell culture assay, or the inhibitory concentration where 50% inhibition in a cell culture is observed (IC₅₀).

An "antibody" when used herein is a protein comprising one or more polypeptides (comprising one or more binding domains, preferably antigen binding domains) substantially or partially encoded by immunoglobulin genes or fragments of immunoglobulin genes. The term "immunoglobulin" (Ig) is used interchangeably with "antibody" herein. When used herein the term "antibody" does not only refer to an immunoglobulin (or intact antibody), but also to a fragment thereof, and encompasses any polypeptide comprising an antigen-binding fragment or an antigen-binding domain. Preferably, the fragment such as Fab, F(ab')₂, Fv, scFv, Fd, dAb, VHH and other antibody fragments that retain antigen-binding function.

The term "downstream process" or purification and/or concentration process relates to a process that comes after the actual production process of the biological product. Biological products are usually produced using a host cell that produces the biological product via fermentation of the host cell. The biological product can be secreted from the host cell into the cultivation medium or remain in the cytosol of the host cell soluble or in form of inclusion bodies (IB). The biological product is then purified in the downstream process which is a purification and /or concentration process comprising different purification and/or concentration steps. Typical purification and/or concentration steps are cell disintegration (using high pressure homogenizers or microparticles or reverse osmosis and the like), IB isolation, IB solubilization, refolding, filtration (dead end filtration, deep bed filtration, membrane filtration, tangential flow filtration, ultrafiltration / diafiltration (UF/DF), and the like), dialysis, chromatography, crystallization, precipitation (e.g. using ammoniumsulfate, potassium chloride, heat and the like), adjustment of pH, salting, extraction (like aqueous two phase extraction), proteolytic cleavages (chemical or enzymatic or autoproteolytic), digestion of RNA using RNAses, ultrafiltration , nanofiltration using nanomembranes and any other purification and/or concentration steps known in the art.

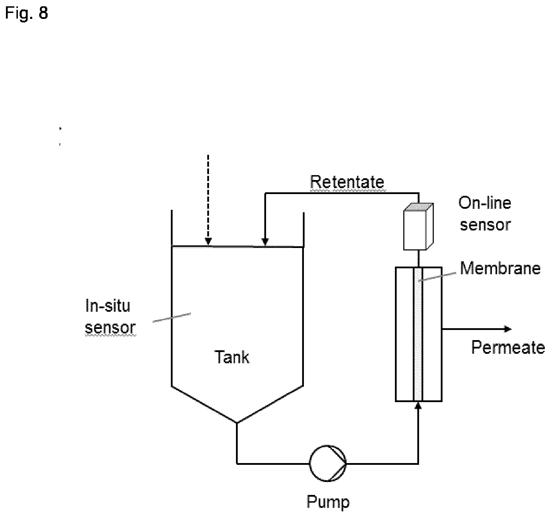

The term "operation unit" as used herein is a system for purification and/or concentration of the biological product. In the context of the present invention, the expression "operation unit" comprises at least one purification unit and/or a concentration unit. The operation unit may comprise one or several chromatography units and/or a filtration unit. It is specifically intended that the operation unit include laboratory and industrial scale units. A column for use in a laboratory scale operation unit may have a scale between 1 ml to 100 ml. Pilot and industrial scale operation units may have a scale from about 100 ml up to about 2000 liters. Further operation units may be units for cell disintegration (e.g.: high pressure homogenizers or stirred tank reactors for use of microparticles or reverse osmosis and the like), IB isolation (e.g.: units comprising stirred tank reactors and centrifuges), IB solubilization (e.g.: units comprising stirred tank reactors, pumps and static mixers), refolding (e.g.: units comprising stirred tank reactors, pumps and static mixers), filtration (e.g.: units as shown in Fig. 8 or comprising filter domes), dialysis, chromatography (e.g.: comprising chromatography columns or units as shown in Fig. 3), crystallization (e.g.: units comprising a stirred tank reactor and a filter nutsche), precipitation (e.g. unit comprising a heat exchanger and/or astirred tank reactor), adjustment of pH (e.g.: unit comprising a stirred tank reactor), salting (e.g.: unit comprising a stirred tank reactor), extraction (eg.: a unit comprising a counter current extractor), proteolytic cleavages (e.g.. unit comprising a stirred tank reactor or an enzyme reactor with immobilized enzymes , e.g. in a column), digestion of RNA using RNAses (e.g.: unit comprising a stirred tank reactors or an enzyme reactor with immobilized enzymes , e.g. in a column) , ultrafiltration , nanofiltration using nanomembranes (both e.g.: unit as shown in Fig. 8) and units for any other purification and/or concentration steps known in the art.

The term "online" as used herein with respect to the methods of the invention refers to direct computer control of a process in real-time. Online monitoring and control can be effected by sensors placed *in situ,* inline, or, in certain cases for slow processes, atline, and respective measurements. These sensors are referred as online sensors. For a measurement to be considered "online", the time in which information about process or material properties is obtained and analysed must be shorter than the time in which these properties change.

The term "atline" as used herein refers to analyses which are characterized by manual or automatic sampling followed by discontinuous sample preparation, measurement and evaluation. The material properties can change during the time between sampling and the availability of the results, so direct process control is only possible for slow processes. Atline analysis is made in close physical and temporal proximity to the process stream and then used for the process control.

The term "inline" as used herein refers to direct determination of process parameters which allow inference of the properties of the process stream. Usually, inline methods are non-destructive. The measurement takes place in the process stream, either the entire process stream is analyzed or a split stream.

The term "offline" as used herein refers to the analysis of the samples removed from the process stream and analysed in a temporally and physically discrete manner.

The term *"in situ"* as used herein refers to measurements / analyses which take place in the operation unit where the purification and/or concentration process takes place, for example within the chromatography or filtration unit.

The term "non-invasive" means that the method or device does not change or disturb the process stream. Thus, for example, a non-invasive *in-situ* sensor would be attached to or be incorporated within the wall of a chromatography column to make its measurements without interfering with the product stream in the column itself.

The term "non-destructive" refers to a sampling method that does not change the properties of the biological product or the sample measured. Thus, for example, the measurement of protein quantity does not destroy the protein or alter its configuration.

The term "sensor" means a device capable of sensing, detecting, measuring, monitoring, determining or quantifying the presence or amount of one or more substances including the physico-chemical and or biophysical characteristics of the product such as e.g. conformation, folding and/or modification of the product, or events and includes, without limitation, mechanical sensors, force and mass sensors, acoustic sensors, chemical sensors, biosensors, electrochemical sensors, optical sensors, electromagnetic sensors, electrical sensors, electronic sensors, optoelectronic sensors and, photodetectors. In the context of the invention the sensors may be selected from the group consisting of UV-VIS-absorption sensors, fluorescence sensors, infrared absorption sensors, light refraction sensors, (optical sensors and or fluorometric sensors), light scattering sensors (static and or dynamic), temperature sensors, SAXS sensors and/or pH, conductivity sensors. The term "sensor" may specifically refer to Attenuated total reflection (ATR) - Fourier transform infrared spectroscopy (FTIR), fluorescence detection, multi angle light scattering (MALS) and/or refractive index detector (RI). In this regard it is important to note that a sensor, such as a fluorescence sensor, refers to a unit as a whole that measures the intended property. Thus the term fluorescence sensor includes all of the emission and excitation sensors necessary to measure the fluorescence of the product. Online sensors are, for example, flow through cells for UV/Vis detectors or pH sensors as used in state of the art chromatography systems for measurements directly in the product stream or FTIR detectors using ATR probes that are directly placed in the fluid to be measured e.g. in the refolding solution in a stirred tank reactor. Online sensors, such as flow thorough cells for ATR-FTIR sensors may be included in the load and the eluate stream of a chromatography unit.

The term "real time" as used herein refers to measuring parameters of a purification or concentration step while purification or concentration is occurring. Real time measurements are performed contemporaneously with the monitored, measured, or observed purification events, as opposed to offline analytics. For example, a real time measurement can comprise the determination of the rate of increase or decrease in the amount of product bound to a matrix. "Real-time" measurements also encompass processes where the change of the properties to be monitored is slower than the response of the analysis. As used herein, "real-time" refers to at least one of the time of occurrence of the associated events, the time of measurement and collection of predetermined data, the time to process the data, and the time of a system response to the events and the environment being simultaneous. In the embodiments described herein, these activities and events occur substantially instantaneously. That means that the associated event occurs within 10 sec, 9 sec, 8 sec, 7 sec, 6 sec, 5 sec, 4 sec, 3 sec, 2 sec or 1 sec of the measurement and collection of the data.

"Real-time release" refers to a test methodology that is able to evaluate the quality of an in-process product, intermediate and/or an end-product, by testing results of raw materials and/or data obtained in a manufacturing process without carrying out end-product testing, and ensuring that the quality is acceptable.

The term "parametric release" refers to the release of a product based on data collected during the manufacturing process to demonstrate that the product meets specific, pre-set parameters, such as e.g. concentration, purity and/or potency. Additionally, the manufacturing process of the desired product can successfully be validated based on process monitoring carried out during manufacturing in order to ensure the desired quality of the product. Based on the obtained data the product could immediately be released for further intended usage.

Thus, parametric or real-time release refers to quality testing that ensures the quality of a product to be obtained on the basis of data obtained during the manufacturing process that influence the quality of the product. Use of real-time release testing eliminates the need for pre-shipment quality testing and therefore advantageously permits immediate shipment after manufacture. In real-time release a product to be obtained finally is judged as satisfying the target quality if the data obtained during the manufacturing process fall within the defined specification range.

The term "controlling" in the context of the methods of monitoring and controlling the purification and/or concentration processes of the invention includes diagnosing the data from the online sensors as well as regulating and optimizing the processes that are being monitored. Controlling can also refer to choosing not to regulate or optimize the process in cases where no action is necessary. In one aspect, controlling can include for example real time purity control of the biological product. Controlling means diagnosing the actual process data values and optionally regulation of the purification and/or concentration process.

The term "regulation" or "regulating" the purification and/or concentration process means the manipulation of a process by measuring the value/values of a certain output process parameter / of certain output process parameters (like concentration, purity and/or potency of a biological product or certain impurities of a biological product), evaluating the difference of the measured output parameter / parameters to a predefined operation value / predefined operation values or ranges thereof by changing one or more input parameters in order to operate again within the values or ranges of the predefined operation value / predefined operation values or ranges thereof if the output parameters deviate from the predefined operation values or ranges. Regulation also means a manipulation of a process or process step depending on a value/values of a certain output process parameter / of certain output process parameters (like concentration, purity and/or potency of a biological product or certain impurities of a biological product) for example as interruption of the process, abandonment of the process, stopping a process step, starting with the next process step, start and stop of eluate collection during a chromatography process, stopping loading a chromatography column during a chromatography process, stopping an enzymatic process such as a proteolytic or autoproteolytic step, addition of further amounts of diafiltration buffer during a diafiltration process, temperature changes during a refolding process, stopping an IB resolubilization step, and the like.

The term "diagnosing" or "diagnose" refers to the evaluation and judgement of the obtained actual process data values corresponding to the respective output of the sensors and/or multivariate process data and/or the actual predicted values of concentration, purity and/or potency of the biological product in order to compare said actual values/data with other batches of the same process and/or to identify if the said actual values/data are within predefined specifications and/or to evaluate if the (desired) purpose of the purification and/or concentration process and/or process step is met (e.g.: but not limited to if certain impurities are separated appropriately / as desired by a certain chromatography step or if the desired concentration of the biological product is reached during an ultrafiltration step or to which extend a biological product is refolded from a non-active conformation to a native, active conformation during a refolding step or to detect the end point (e.g.: 60% of the biological product is refolded) of a refolding process or to detect the end point of a crystallization, precipitation, salting, ptoteolytic or autoproteolytic process or to evaluate if the biological product characteristics are changed during hold times and the like) and/or to identify and/or eliminate special causes of variation, and/or to optimize the purification and/or concentration process and/or, as final result, release the biological product. Such a process diagnosis leads to the elimination of common causes of variation, and eventually to process improvement.

It is to be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the methods and uses described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "consisting", "consisting of" and "consisting essentially of" may be replaced with either of the other two terms.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

As described herein, "preferred embodiment" means "preferred embodiment of the present invention". Likewise, as described herein, "various embodiments" and "another embodiment" means "various embodiments of the present invention" and "another embodiment of the present invention".

The word "about" as used herein refers to a value being within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects according to the invention. In this context "about" may refer to a range above and/or below of up to 10%. The word "about" refers in some embodiments to a range above and below a certain value that is up to 5%, such as up to up to 2%, up to 1%, or up to 0.5 % above or below that value. In one embodiment "about" refers to a range up to 0.1 % above and below a given value.

Several documents are cited throughout the text of this disclosure. To the extent the material cited herein contradicts or is inconsistent with this specification, the specification will supersede any such material. Nothing herein is to be construed as an admission that the invention is not entitled to antedate such disclosure by virtue of prior invention.

The following detailed description includes information that may be useful in understanding the present invention. It is not an admission that any of the information provided herein is prior art or relevant to the presently claimed inventions, or that any publication specifically or implicitly referenced is prior art.

### Detailed description of the invention

The present invention relates to a computer based method for monitoring and controlling the purification and/or concentration process of a biological product in real time. The method relies on the use of multivariate statistical analysis to provide information on the concentration, purity and/or potency of the biological product in real-time by diagnosing the data from at least two online sensors and feeding this data to a predictive model obtained previously from online and offline data. In addition, the invention provides a device for use in these methods.

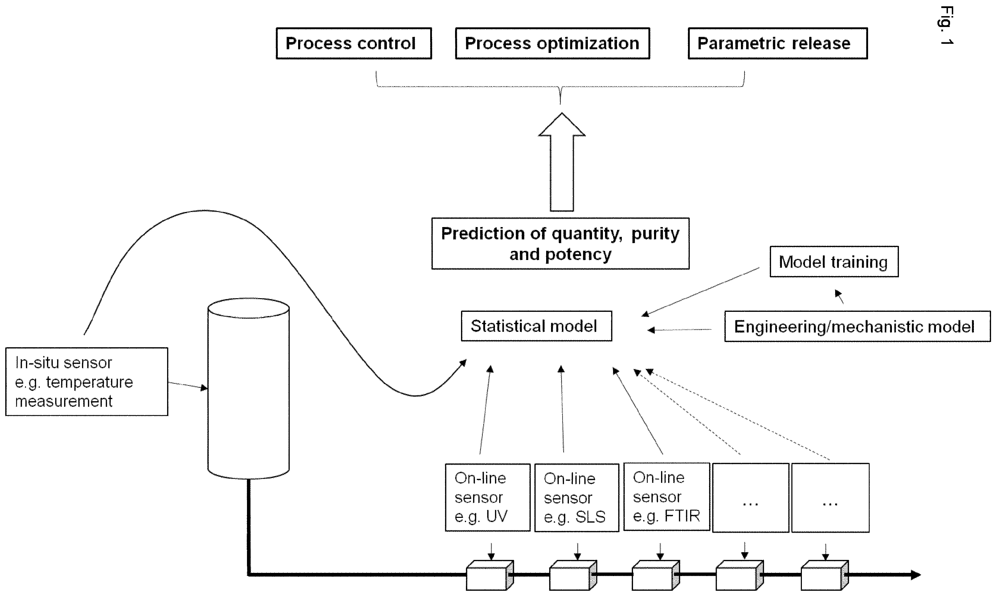

First, in order to obtain a predicative model for a specific property of the product, such as concentration, purity or potency, both online and offline data was collected for the intended biological product. As can be seen from the process overview of Figure 1, data of online and *in situ* monitors can be evaluated in context with offline data for model generation and training. Via statistical methods a statistical model is established which enables prediction of purity, potency and quantity. Prediction of named attributes allows parametric or real time release, process optimization and process control.

### Predictive Model Building

As described in detail in Example 7, for the predictive models used in the Examples, online data from the UV-VIS, Conductivity, Pressure, pH, MALS and RI devices (with p = 14 variables in total), ATR-FTIR spectra (resolution approximately 2 cm⁻¹ resulting in p = 1427 predictors) as well as fluorescence emission spectra at 7 excitation wavelengths (resolution about 0.3 nm) giving in total 14366 fluorescence variables was collected. Depending on the predictor sets and the response, variable data from 7 to 14 chromatographic runs were used for model building. After this a time alignment step was performed - averages were calculated for each online variable corresponding to the time frame of each offline fraction considering the known time delay between several devices. The results were then obtained by STAR (structured additive regression) models in combination with boosting as a variable selection technique (R package *mboost*)*.* Parameter optimization and model selection was performed on autoscaled data (i.e. from each predictor the mean is subtracted and divided by its standard deviation) via cross validation (data from each run are left out once and predicted by a model based on the data of several other runs). The model quality was measured with the cross-validated root-mean-squared error (RMSE). In this way models for the different properties of the intended biological product can be established.

In Example 7, models for Quantity (Concentration), Purity (Host Cell Protein (HCP), double-stranded DNA (dsDNA), Monomer and High Molecular Weight HMW impurity concentrations) and Potency (KD value) were established for the biological product FGF2.

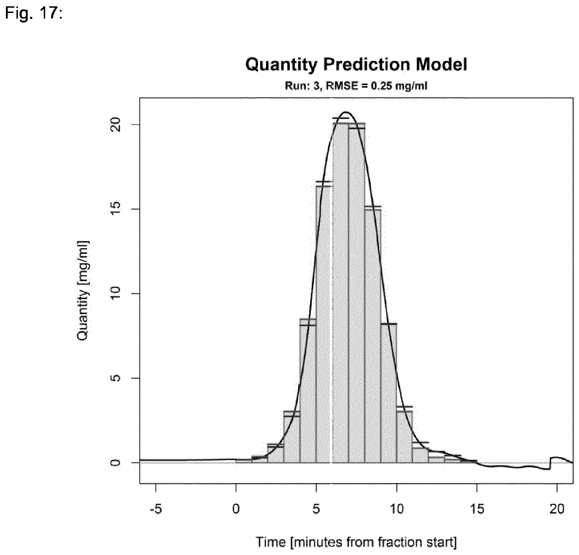

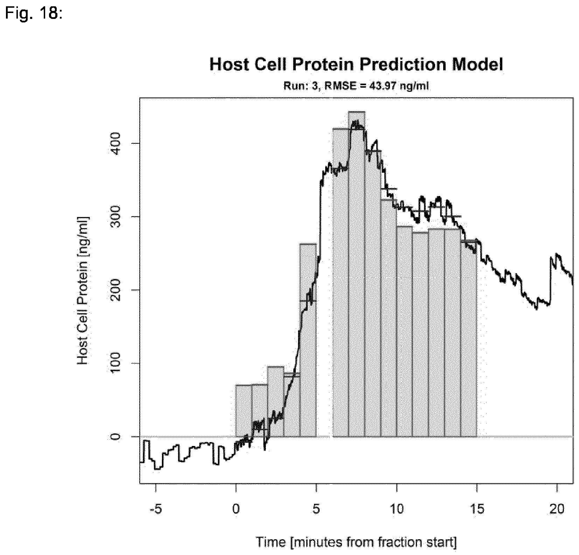

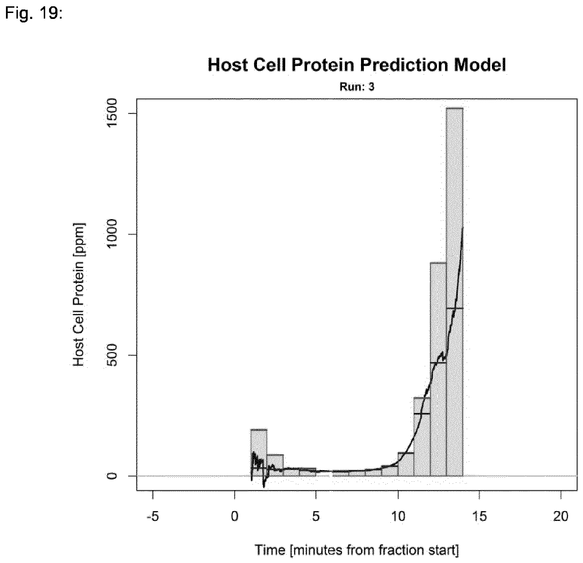

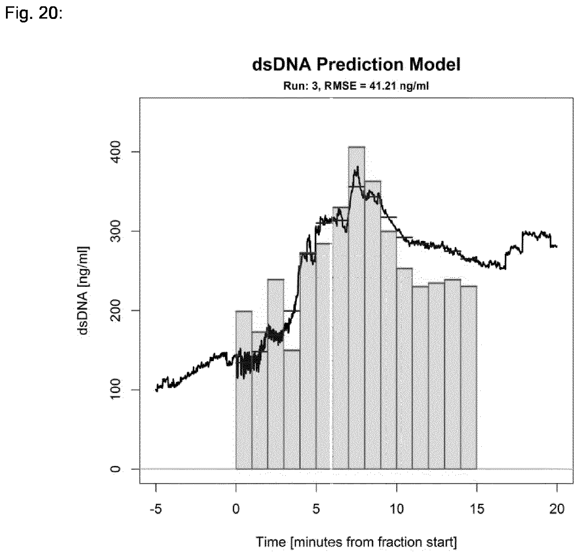

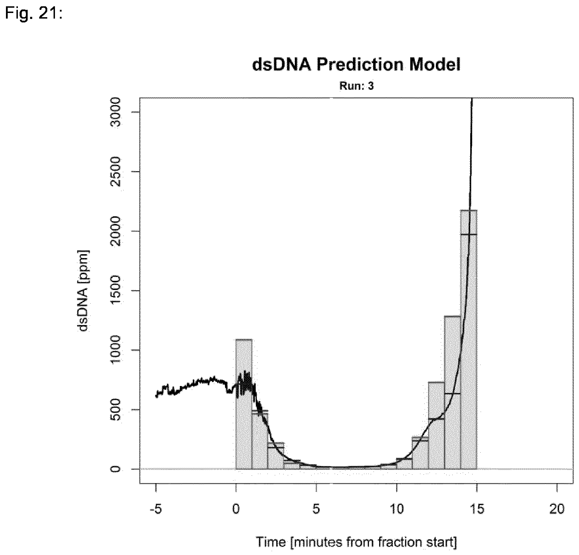

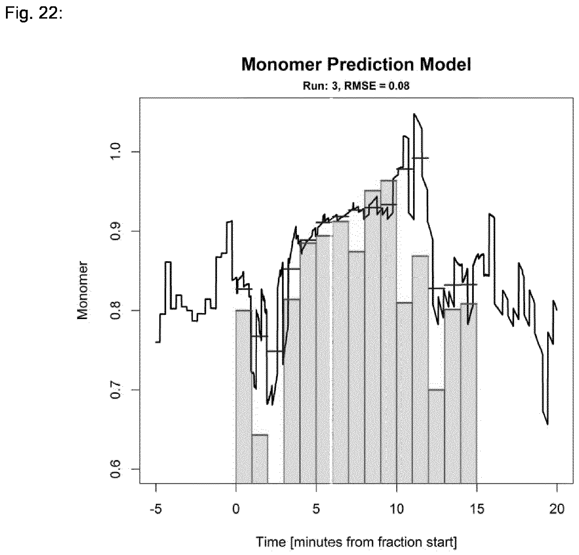

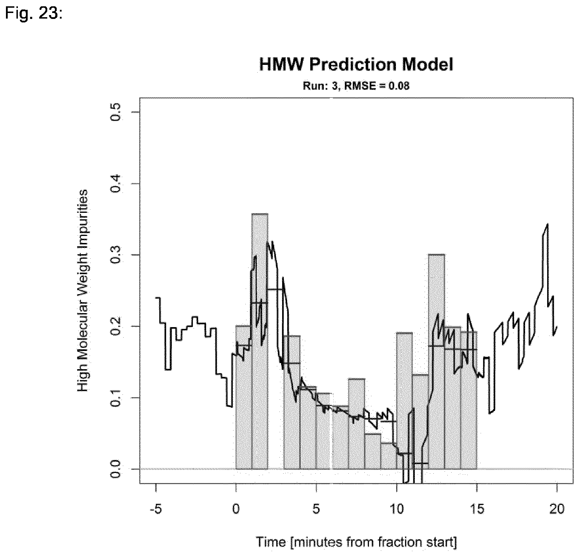

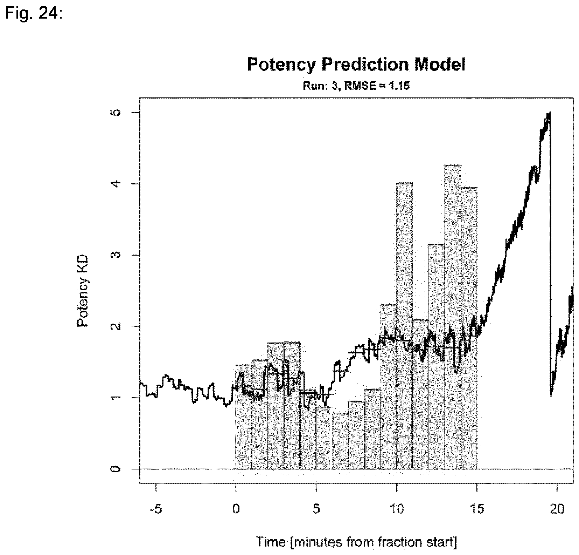

Exemplary modeling results and a comparison to the corresponding offline values for the subsequent target variables are shown in the following Figures:
1. Protein quantity (concentration) in mg/ml (Fig. 17)
2. Host cell protein concentration in ng/ml and relative to the estimated protein quantity in ppm (Fig. 18 and 19)
3. Double-stranded DNA (dsDNA) concentration in ng/ml and relative to the estimated protein quantity in ppm (Figs. 20 and 21)
4. Monomer and high molecular weight impurities in % (Figs. 22 and 23)
5. Potency expressed by the KD value (Fig. 24).
In these figures, actually measured offline values for the targets are given as bars, the model predictions based on online data on a time grid of 1 second are depicted as curves and these predictions averaged over each offline fraction are shown as horizontal lines. Results are given for a single chromatographic run, with prediction errors similar to the overall prediction error (averaged over all runs). The horizontal axis represents time (in *minutes)* with the origin placed at the start of the first offline fraction.

For the model building/multivariate statistics offline analytics has been used. In this regard, any offline method for measuring concentration, purity and potency of the biological product can be used. For example, for measurement of concentration (quantity) Reverse Phase-HPLC, Affinity-HPLC, and ELISA can be used. Specifically, for determination of quantity/concentration in the predictive models developed in the examples, reverse phase HPLC was used to determine FGF2 concentration. For the determination of antibody concentration, Protein A columns were used in bind-elute mode to reveal total antibody concentration. For analysis of purity, size exclusion chromatography to determine monomer content and high and low molecular weight impurities, LAL endotoxin test, host cell protein ELISA, and dsDNA quantification via Picogreen assay can be used. For analysis of potency, a biosensor based on surface plasmon resonance, proliferation assays for testing the bioactivity of biological products and cell culture potency assays can be used.

In addition, the online data obtained from the online sensors for each individual run for a given biological product is evaluated in the context of the offline data for a combined prediction of concentration, purity and potency using multivariate statistical analysis. Multivariate analysis (MVA) is based on the statistical principle of multivariate statistics, which involves observation and analysis of more than one statistical variable at a time.

In one embodiment of the invention, the multivariate statistical analysis used in the predictive model building is based on machine learning techniques.

Machine learning is a method of data analysis that automates analytical model building. Machine learning is a subfield of computer science that evolved from the study of pattern recognition and computational learning theory in artificial intelligence. Machine learning explores the study and construction of algorithms that can learn from and make predictions on data. Such algorithms operate by building a model from example inputs in order to make data-driven predictions or decisions rather than following strictly static program instructions. Machine learning is closely related to computational statistics and has strong ties to mathematical optimization.

Machine learning is closely related to statistics and often employs the same methods and overlaps significantly. In the following, different methods of multivariate data analysis and machine learning are summarized briefly. Decision tree ensembles, also referred to as random forests (RF), are useful for feature selection in addition to being effective classifiers. One approach to dimensionality reduction is to generate a large and carefully constructed set of trees against a target attribute and then use each attributes usage statistics to find the most informative subset of features.

An artificial neural network (ANN) learning algorithm, usually called "neural network" (NN), is a learning algorithm that is inspired by the structure and functional aspects of biological neural networks. Computations are structured in terms of an interconnected group of artificial neurons, processing information using a connectionist approach to computation.

Support vector machines (SVM) are a set of related supervised learning methods used for classification and regression. Given a set of training examples, each marked as belonging to one of two categories, an SVM training algorithm builds a model that predicts whether a new example falls into one category or the other.

Multivariate adaptive regression splines (MARS) is a non-parametric regression technique, which builds a regression model as a weighted sum of basis functions, such as constants, hinge functions or products of two or more hinge functions. The latter allow for the modeling of interactions between predictor variables.

Ridge regression, Lasso (least absolute shrinkage and selection operator) and elastic net are regression techniques performing automatic variable selection and regularization (parameter shrinkage) increasing the interpretability and in many cases also the predictive performance of a prediction model.

Least angle regression (LARS) is a regression method particularly suited for high-dimensional data and similar to stepwise regression.

Structured additive regression (STAR) models are an extension of linear models also incorporating smooth effects of covariates as well as interaction effects between predictors.

Principal component analysis (PCA) is an unsupervised multivariate statistical method which transforms a set of (potentially many) variables into a smaller set of uncorrelated variables, called principal components (PCs). This transformation is performed in such a way that the direction of each PC accounts for the highest possible variance in the data set, while being orthogonal to several previous PCs. Usually, the first few PCs cover a large amount of the total variability (information) of the original data set and hence lead to a significant data reduction.

Partial least squares (PLS) regression is a multivariate regression technique for analysis of systems of independent and response variables. PLS can also relate the set of independent variables to a set of multiple dependent (response) variables. Partial least squares discriminant analysis (PLS-DA) is a variant used when the Y is categorical.

Multivariate curve resolution (MCR) resolves time evolving data such as chromatographic data into pure spectra and the corresponding concentration, purity and potency profiles.

The machine learning technique used in predictive model building is selected from partial least squares regression, principal component regression, random forest, neural networks, structured additive regression, multivariate adaptive regression splines, Ridge regression, lasso, elastic net, least angle regression or support vector machines as described above.

In addition engineering/mechanistic models can be used in combination with the obtained data for model building. For instance, the loading of a chromatography column packed with porous beads can be predicted with several mathematical models. The equilibrium binding capacity can be estimated by several methods such as stirred tank method, shallow bed or small scale chromatography experiments. The binding kinetics and other parameters such as the effective diffusivity can also be obtained from these experiments. After having estimated these parameters mathematical models using rate equations for description of mass transfer in spherical adsorbent particles can be applied in order to predict the breakthrough curve and/or the extent of loading. Such mathematical models are pore diffusion, solid diffusion, parallel pore and solid diffusion, film diffusion, parallel film and pore diffusion or diffusion bi-dispersed particles. The migration of the product from loading as a function of column length and time is computed using these models. The same can be done for elution of a peak from a chromatography column. If required the predicted loading front or elution profile can be convoluted with zone spreading happening before and after the column.

As can be seen from the above example, the combination of engineering/mechanistic models with statistical analysis sharpens the prediction.

In a further option for model building, statistical methods may combine mechanistic models such as pore diffusion models/ solid diffusion models, film diffusion models with extra column band spreading to meet the physical reality. The multi component situation, which requires a lot of experiments, for parameter estimation has been solved by model training. The model takes column, flow uniformity, and band spreading by adsorption/desorption mechanisms into account based on the specific operation unit used. In cases when pore diffusion is the dominating process, analytical solutions and simple numerical solutions have been used to predict the composition of the process stream. This enhances speed and allows computer aided exploration of operating ranges.

An important aspect of the invention after conducting the model training, no further calibration steps with respect to the biological product are required for the sensors so that the methods of the invention can be applied to the intended biological product without changing the setup.

Once the model is established for a give biological product, the method of the invention can be used without further adjustment of the predicative model and without obtaining offline data. In addition, as mentioned above, the online sensors, which were used for predictive model building, are also used to obtain the data values for monitoring and controlling the processes of the invention and do not need to be calibrated with respect to the biological product. This allows for real-time monitoring and controlling of the purification and/or concentration process of the biological product as well as the monitoring and control of real-time or parametric release of the product.

### Methods of controlling and monitoring the purification and/or concentration processes

Once the predictive model has been established for a biological product, the method of the invention can be used to monitor and control the purification and/or concentration of the biological product. Thus the invention also relates to an improved method for manufacturing a biological product of interest comprising the steps of expressing said product via any conventional/known in the art expression technologies, purifying the expression product by methods also in principle known in the art, but additionally applying any of the methods of the invention, and optionally finishing, e.g. formulating the purified expression product, in order to obtain a product of interest ready to use or commercialize.

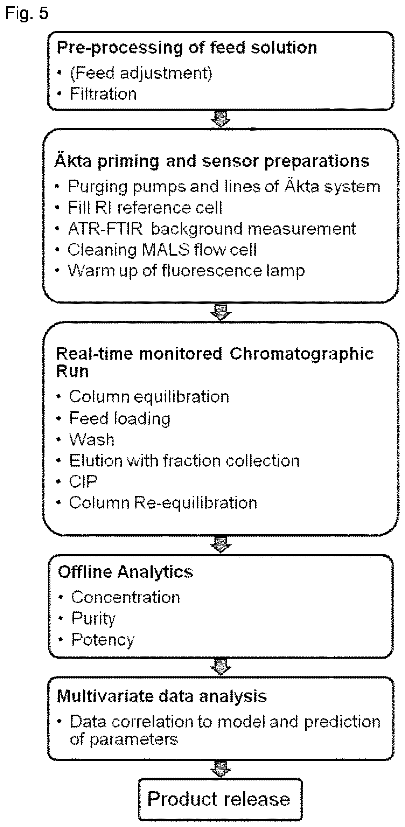

When Implementing a real time purity control the set-up and operation of e.g.: a chromatographic separation, a protein refolding step or a membrane separation will not differ substantially compared to a conventional set-up, since this will imply only the installation of online detectors into the established system. An example of a possible setup is shown in Figure 3. As can be seen from Figure 5, starting with preprocessing, preparation of sensors/chromatography system, the chromatographic run is started. The predictive model enables prediction of purity, potency and concentration (quantity) and thus allows for immediate product release.

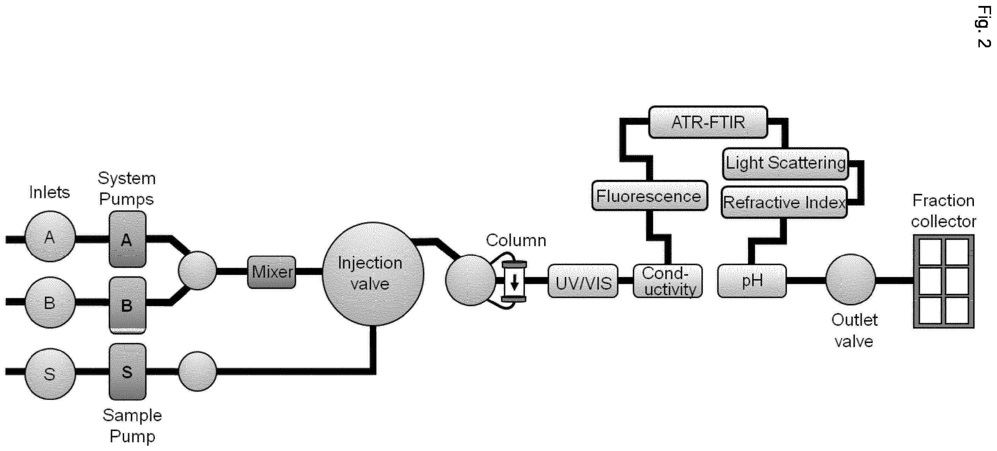

The sequence of operations of a real-time monitored downstream process can be seen in Fig. 5. As can be seen here, the target protein is obtained from a fermentation process or a prior downstream unit operation. Depending on the expression host, the process solution containing target protein is preprocessed (e.g. 0.2 µm dead end filtration before chromatographic step, feed adjusted such as pH value or conductivity for loading step, solubilization of inclusion bodies). In the present examples below, chromatographic runs were performed on an Äkta Pure 25 system (GE Healthcare, Sweden), which is equipped with its standard sensors for UV-VIS, conductivity, pH and pressure. Specifically, the Äkta Pure 25 system comprises the multi-wavelength UV detector U9-M (UV-VIS, 280nm, 260nm, 214nm - max. 3 wavelengths simultaneously in the range 190 - 700nm), the conductivity probe C9 and the pH probe V9-pH (pH 0-14). The system is controlled by UNICORN software version 6.4. As can be seen in Figure 2, in the operating unit used in the examples, online sensors are integrated after the chromatography column. A flow diagram of the chromatography system equipped with additional online sensors is shown in Fig. 2. In the setup of this figure, the mid infrared spectrometer MATRIX-FM (Bruker, USA) based on attenuated total reflection (ATR) was chosen to record Fourier transform infrared spectroscopy (FTIR) spectra. For fluorescence detection a set-up comprising of laser-induced xenon lamp EQ-99XFC LDLS (Energetiq, USA), a fiber optic multiplexer (Avantes, Netherlands), a flow cell (FIAlab Instruments, USA) and the spectrometer AvaSpec-ULS-TEC with 600 L/mm grating (Avantes, Netherlands) was assembled. This fluorescence device enabled excitation light of 7 different wavelengths (Pos. 1-reference, Pos. 2 - 265 nm ± 10 nm, Pos. 3 - 280 nm ± 10 nm, Pos. 4 - 300 nm ± 40 nm, Pos. 5 - 340 nm ± 10 nm, Pos. 6 - 289 nm ± 10 nm, Pos. 7 - 300 nm ± 10 nm, Pos. 8 - 400 nm ± 10 nm) and measurements of whole emission spectra (236 - 795 nm). Additionally, the multi angle light scattering (MALS) detector miniDAWN TREOS (Wyatt, USA) as well as the differential refractive index detector Optilab T-rEX (Wyatt), differential RI in the range of -0.0047 - +0.0047 RIU were applied. All detectors were connected in series, taking into account the peak delay as well as band broadening effects.

According to the invention there is provided a computer based method for monitoring and controlling the purification and/or concentration process or parts thereof of a biologically produced product which comprises at least one operation unit, comprising the steps of
a) applying at least two independent online sensors at said operation unit,
b) monitoring process data in real time,
c) and/or obtaining data from in-situ measurements or analysis
d) obtaining a plurality of process data values corresponding to the respective output of said sensors;
e) performing a model based multivariate statistical analysis on said process data values for the prediction of concentration, purity and/or potency of the biological product;
f) diagnosing the actual process data values;
g) optionally performing process control and/or process optimization, and
h) optionally parametric or real time release of the biological product as in-process, intermediate and/or end product).

In one embodiment of the invention the operation unit is a purification unit and comprises a chromatography unit and/or filtration unit.

Embodiments of the invention include application of the methods described herein to any type of purification or concentration method, preferably to chromatography and filtration methods wherein the operation unit is a chromatography or a filtration unit.

Suitable chromatography methods include, for example but without limitation:, liquid chromatography such as high performance liquid chromatography; affinity chromatography; supercritical fluid chromatography; ion exchange chromatography; size-exclusion chromatography; reversed phase chromatography; two-dimensional chromatography; fast protein (FPLC) chromatography; countercurrent chromatography; chiral chromatography; aqueous normal phase (ANP) chromatography; mixed mode chromatography; pseudo-affinity chromatography; hydrophobic interaction chromatography, and multi-modal resin chromatography.

. The chromatography unit may be operated in isocratic, linear, segmented and/or step gradient elution, in bind/elute or flow through mode.

In filtration methods, the operation unit is a filtration unit and can be selected from the group consisting of ultrafiltration, microfiltration, nanofiltration, depth filtration. The filtration unit may be operated as tangential flow filtration, dead end filtration, or filtration through absolute pore size membranes.

In order to monitor process data and/or obtain a plurality of process data values in real time, a plurality of online sensors is implemented in the operation unit.

In one embodiment of the invention, at least 2, 3, 4, 5, 6, 7, 8, 9, 10, 11, 12 or more online sensors are implemented in the operation unit. The sensors may directly be placed in the product stream or may be non-invasive *in-situ* sensors as defined above.

A sensor acquires a physical quantity and converts it into a signal suitable for processing (e.g. optical, electrical, mechanical, thermal). As defined above, the sensors may be electrical sensors, electro-mechanical sensors, electronic sensors, transducers, resistive sensors, capacitive sensors, electromagnetic sensors, switches, optical sensors, magnetic sensors, and/or inductive sensors, temperature sensors. Suitable sensing methods include measurement of fluorescence, UV-adsorption, infra-red, electrical or electrochemical impedance, pH, conductivity, static dynamic light scattering, dynamic light scattering refractive index, temperature.

In one embodiment of the invention, the sensors are selected from the group consisting of light scattering sensors such as multi angle light scattering (MALS), UV-VIS-absorption sensors, fluorescence excitation and emission sensors, infrared adsorption sensors such as Attenuated total reflection - Fourier transform infrared spectroscopy (ATR-FTIR), light refraction sensors such as refractive index sensors (RI) (optical sensors and or fluorometric sensors), and small angle X-ray scattering sensors (SAXS), conductivity sensors, temperature sensors and/or pH.

In one embodiment of the invention the sensors are connected in series as can be seen in figures 1 and 3. In an alternate embodiment, the sensors are integrated into the operation unit and are non-invasive *in situ* sensors that measure within the process stream.

In one embodiment of the invention, UV-VIS absorption, conductivity data, pH values, pressure data, fluorescence excitation and emission, infrared adsorption (ATR-FTIR), light refraction (RI), and static light scattering (MALS) are simultaneously acquired from the process stream.

In addition temperature, SAXS, ATR-FTIR, pH, conductivity and reduction-oxidation (redox) may be measured *in situ* during the purification step such as a chromatography and/or any other purification step. The temperature changes may be marginal that means in the range of tenths of a degree.

By a special algorithm the quantity of the product, the purity, and/or the potency are inferred for the data values obtained from the sensors as will be explained below. The method is so fast that it is suited for in process control in downstream processing and real time or parametric release of therapeutic products in biopharmaceutical manufacturing.

### Multivariate Statistical Analysis of the data

For the multivariate statistical analysis used in the methods of the invention, the online data obtained from the online sensors for each individual run for a given biological product is imported into a computer database, evaluated by the predictive model as described above and diagnosed to allow for real-time monitoring of the concentration, purity and/or potency of the biological product.

The plurality of process data obtained from the online sensors and stored in a database. The data is statistically and mathematically evaluated. Data sets for the biologically produced compound are selected, extracted and imported into a statistical computing environment, where they are evaluated using Multivariate analysis as described above for the predictive model.

### Devices of the Invention

Also provided is a device comprising an operation unit for purification and/or concentration of a biological product, wherein the operation unit comprises a chromatography and/or a filtration unit, wherein the chromatography or filtration unit comprises at least one non-invasive *in-situ* sensor and wherein the sensor is connected to a computer where the data values collected can be stored in a database and/or diagnosed.

A schematic overview of the intended device is shown in Figure 3. The schematic drawing shows the online monitoring device comprising of a pressure sensor, a UV-VIS sensor, a conductivity sensor, and a pH sensor, which are standard detectors of the purification unit, as well as an ATR-FTIR sensor, a fluorescence sensor, a MALS sensor and a RI sensor for inline measurements and a temperature sensor, a redox sensor, an ATR-FTIR sensor, and a small angle x-ray scattering sensors (SAXS) sensor installed for non-invasive *in situ* measurements. The dashed arrow indicates possible real-time control of the process based on the statistical model.

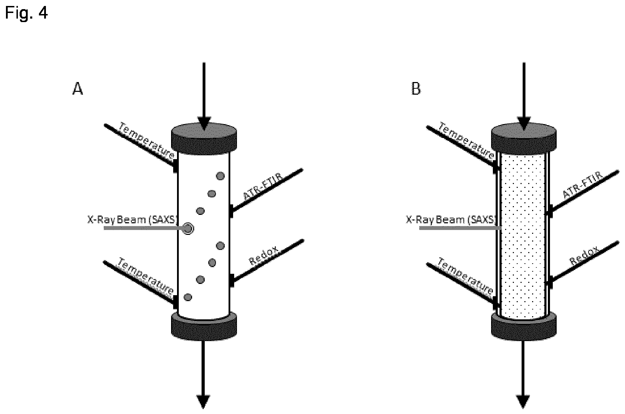

In the device of the invention, the non-invasive *in-situ* sensors can be selected from the group consisting of temperature sensors, SAXS, pH sensors, conductivity sensors, ATR-FTIR sensors and redox sensors. As can be seen from the more detailed diagram in Figure 4, one or several *in situ* online sensors can be integrated into the chromatography or filtration unit. In this schematic drawing, the chromatography column is equipped with non-invasive *in situ sensor*s: two temperature sensors, a SAXS sensor, an ATR-FTIR sensor, and a redox sensor exemplarily. A) Exterior and B) interior view.

In addition, as can be seen from Figure 3, the device can further comprise at least one online sensor selected from the group consisting of multi-angle light scattering sensors (MALS), UV-VIS absorption sensors, fluorescence sensors, infrared absorption sensors (IR), attenuated total reflection-fourier transform infrared spectroscopy sensors (ATR-FTIR), light refractive index (RI) sensors , pH sensors, temperature sensors, conductivity sensors, pressure sensors, small angle x-ray scattering (SAXS) sensors and redox sensors. The online sensors can be connected in series as shown.

In a preferred device, the chromatography or purification unit of the device comprises non-invasive *in situ sensors* for SAXS, ATR-FTIR, temperature and redox as well as additional online sensors for fluorescence, RI, MALLS and UV-VIS.

The purification or filtration unit of the device can be at laboratory or at industrial scale. A column for use in a laboratory scale device may have a scale between 1 ml to 100 ml. Pilot and industrial scale devices may have a scale from about 100 ml up to about 2000 liters.

### Applications of the methods and devices of the invention

The present invention provides for substantial economic improvements of downstream processes. For example, it allows the comparison of different batches at all stages of the bioprocessing system from end of fermentation product to final purified biological product such as a biopharmaceutical.

A further embodiment of the invention therefore relates to the method as described herein, wherein the observation of a deviation pattern in the data indicates the malfunctioning of the purification step. Upon detecting of such a deviation pattern the process parameters could be regulated accordingly or the process is interrupted. Such a deviation could be product aggregation or no elution of target protein, protein truncation, complexation of protein and host cell impurity, precipitation of target protein, precipitation of host cell protein and combinations thereof.

A further embodiment of the invention relates to the method as described herein, wherein upon detection of certain discrepancies between model predictions and target values or ranges, an adjustment of the purification step is executed based on the result of said measurement and evaluation. For example, when the protein is eluted from a chromatography column, collection of the product is only started when the target values or ranges for purity, potency and/or concentration are reached. Collection is stopped when the target values are not met. Based on the results further downstream unit operations can be adapted. For example in a diafiltration step, higher volume can be used to remove more host cell protein (HCP) or double-stranded DNA (dsDNA) or a concentration step can be performed if target concentration is not reached.

A further embodiment of the invention relates to the method as described herein, wherein the purification process or parts thereof are optimized with regard to any one or a combination of peak collection, correct collection of the biological product after refolding, filtration or precipitation, product quality, economy, environmental aspects, energy consumption, and process equipment maintenance. Thus for a biological product, the composition of the eluted peak in regard to purity, quantity and potency is known. In the next experiment process parameters are modified and the process is repeated until the target values are obtained.

The present invention provides for monitoring and controlling the concentration, purity and/or potency of the biological product in real time. For example, it allows the determination of the concentration, purity and/or potency of the final purified product. Based on the obtained data and the release criteria the product could be further used as drug substance for the manufacture of the final drug formulation.

One embodiment of the invention relates to a method as described herein, wherein the biological product is a nucleic acid molecule, for example but not limited to antisense nucleic acids, plasmid DNA, mRNA, microRNA, dsRNA, siRNA, a heterologous protein, preferably selected from therapeutic proteins, enzymes and peptides, protein antibiotics, fusion proteins, carbohydrate-protein conjugates, structural proteins, regulatory proteins, vaccines and vaccine like proteins or particles, process enzymes, growth factors, hormones and cytokines, or a metabolite of said biological product. Alternatively, the biologically produced product is a carbohydrate, lipid, organic small molecule, non-organic small molecule, virus, liposome, antibody and hybrid or variant form of any such compounds.

A further embodiment of the invention relates to the use of a method as described herein for establishing a control or release algorithm for the specific biological product. The obtained data are subject to a multivariate statistical analysis in order to determine when to release the biological product.

The invention helps to better understand bioprocesses for the manufacturing of originator, biosimilar or biobetter compounds. In particular, at the stage of final purified product, additional information on the biological product can be obtained in real time.

Based on the model based control/release algorithm for the biological product a method for a comparison between the therapeutic product of an originator and biosimilars or biobetters is also provided.

In another aspect, for important process steps such as chromatography and membrane filtration methodology, the invention helps to predict the performance of material and surface properties. Aging of material, for example, by incomplete regeneration or storage can be monitored and possibly breakdown predicted. Therefore, a further embodiment of the invention relates to predicting the life time of process material.

The invention will help to provide faster access to critical process parameters. By combination of experimental data and statistical models, process parameters are varied and the concentration, purity and/or potency are indirectly obtained by online monitoring. In a preferred embodiment the monitoring is performed at least partially *in situ.* So a relevant operating range can be developed much faster and can be explored in much greater detail. In principle, it is possible to permutate unit operations and to see the effect of the serial connection of unit operations, when at least one step is out of range. Theoretically and practically it is possible, when a single unit operation is out of range, that the entire sequence may still lead to an acceptable product. In such a case the operating space and flexibility of the process is enlarged.

Thus, a further embodiment of the invention relates to faster access to critical process parameters and stress parameters on product quality. Upon analysis of the obtained data an operator may intervene into the process in order to correct malfunctioning.

In one embodiment of the invention a model and software package to control product concentration, purity, and/or potency in real time is provided.

The methods as described herein help to improve yield in downstream processing by more accurate collection of peak fractions, correct collection of material after refolding, filtration or precipitation. Such a tightly controlled downstream process leads to reduced buffer consumption and thus reduced cost of goods.

One further economic improvement is achieved by reduction of batch failures. Batch failures can be recognized at a very early stage and processes can be terminated if indicated and thus avoiding unnecessary expenditure of processing and analyzing a failed batch. In general, using on-line monitoring strategies, processes become more robust.

The significant portion of economic improvement will come from radical reduction of expenditures for in process control and end control.

The economic benefits are achieved by acceleration through *in situ* monitoring and control because feed streams are characterized and the strategy can be generally adjusted.

The methods as described herein also allow the comparison of different batches, at all stages of the bioprocessing system from the end of the fermentation process to the release of the final purified biological product.

Even with existing processes and products, the developed methodology allows a direct comparison feed stream, intermediates and products at all stages. This concept is the basis for a real time release or parametric release in connection with other offline methodology performed after release such as sterility tests. Batch to batch variability is readily observed and adds to improved documentation and validation.

The method helps to document that process deviation did not influence quality of the biological product and eases validation efforts and development of process parameters.

The following items further provide specific aspects of the disclosure, and specific embodiments to practice the teachings provided herein:
1. A computer based method for monitoring and/or controlling the purification process or parts thereof of a biological product which comprises at least one purification unit, comprising the steps of
   a) applying at least two independent online sensors at said purification unit,
   b) monitoring process data in real time,
   c) obtaining a plurality of process data values corresponding to the respective output of said sensors;
   d) performing a model based multivariate statistical analysis and mathematical models on said process data values for the prediction of concentration, purity and/or potency of the biological product;
   e) diagnosing the actual process data values;
   f) optionally performing process control and/or process optimization, and
   g) optionally parametric or real time release of the biological product.
2. The method according to item 1, wherein the purification unit comprises a chromatography unit and/or filtration unit.
3. The method according to item 2, wherein the chromatography unit is selected from the group consisting of ion exchange chromatography, affinity chromatography, size exclusion chromatography, reversed phase chromatography, hydrophobic interaction chromatography, multi-modal resin chromatography, operated in isocratic, linear, segmented and/ or step gradient elution in bind/elute or flow through mode and the filtration unit is selected from the group consisting of ultrafiltration, microfiltration, nanofiltration, depth filtration, operated in tangential flow filtration, dead end filtration, filtration through absolute pore size membranes.
4. The method according to any one of items 1 to 3, wherein the online sensors are selected from the group of light scattering sensors, UV-adsorption sensors, fluorescence excitation and emission sensors, infrared adsorption sensors, light refraction sensors, (optical sensors and or fluorometric sensors), and/or pH, UV-VIS, conductivity sensors.
5. The method according to any one of items 1 to 4, wherein the sensors are connected in series.
6. The method according to any one of items 1 to 5, wherein temperature changes are measured during the purification process *in situ.*
7. The method according to any one of items 1 to 6, wherein said plurality of process data values is stored in a database.
8. The method according to any one of items 1 to 7, wherein data sets for the biological product are selected, extracted and imported into a statistical or technical computing environment.
9. The method according to any one of items 1 to 8, wherein the performed multivariate statistical analysis is based on machine learning techniques.
10. The method according to item 9, wherein the machine learning technique is selected from partial least squares regression, principal component regression, random forest, neural networks, structured additive regression, multivariate adaptive regression splines, Ridge regression, lasso, elastic net, least angle regression, support vector machines or others.
11. The method according to any one of items 1 to 10, wherein the online parameters are evaluated in context with off line parameters.
12. The method according to any one of items 1 to 11, wherein observation of a deviation pattern indicates the malfunctioning of the purification step.
13. The method according to item 12, wherein upon detection of certain discrepancies between model predictions and target values or ranges, an adjustment of the purification step is executed based on the result of said measurement and evaluation.
14. The method according to any one of items 1 to 13, wherein the process is optimized with regard to any one or a combination of peak collection, correct collection of the biological product after refolding, filtration or precipitation, product quality, economy, environmental aspects, energy consumption, and process equipment maintenance.
15. The method according to any one of items 1 to 14, wherein concentration, purity and potency of the biological product are monitored and controlled in real time.
16. The method according to any one of items 1 to 15, wherein the process is a continuous, semi-continuous process, or batch process.
17. The method according to any one of items 1 to 16, wherein no calibration step is required for the online sensors.
18. The method according to any one of items 1 to 17, wherein said biological product is a nucleic acid molecule, a heterologous protein, preferably selected from therapeutic proteins, enzymes and peptides, protein antibiotics, fusion proteins, carbohydrate - protein conjugates, structural proteins, regulatory proteins, vaccines and vaccine like proteins or particles, process enzymes, growth factors, hormones and cytokines, or a metabolite of said biological product.
19. Use of the method according to any one of items 1 to 18 for establishing a model based control/release algorithm for the biological product.
20. Use of the method according to any one of items 1 to 18 for monitoring in real time the concentration, the purity and the potency of the biological product.
21. A method for producing a biological product-of-interest comprising the steps of culturing an organism capable of producing said product of interest; purifying said thereby obtained product of interest, wherein purification of said product-of-interest is monitored by a method according to any one of items 1 to 18, and optionally further processing, sterilizing, finishing, formulating, and the like, the obtained product-of-interest to yield a product ready for use or commercialization.

### General Settings/specifications of online sensors for a real-time monitored purification process in the Examples

Sequence of operations of real-time monitored downstream process can be seen in Fig. 1.

The target protein is obtained from a fermentation process or a prior downstream unit operation. Depending on the expression host, the process solution containing target protein is preprocessed (e.g. 0.2 µm dead end filtration before chromatographic step, feed adjusted such as pH value or conductivity for loading step, solubilization of inclusion bodies).

Chromatographic runs are performed on an Äkta Pure 25 system (GE Healthcare, Sweden), which are equipped with its standard sensors. Flow diagram of the chromatography system equipped with additional online sensors is listed in Fig. 2.

This comprises the multi-wavelength UV detector U9-M (UV-VIS, 280 nm, 260 nm, 214 nm - max. 3 wavelengths simultaneously in the range 190 - 700 nm), the conductivity probe C9 and the pH probe V9-pH (pH 0-14). The system is controlled by UNICORN software version 6.4. The mid infrared spectrometer MATRIX-FM (Bruker, USA) based on attenuated total reflection (ATR) is chosen to record Fourier transform infrared spectroscopy (FTIR) spectra. For fluorescence detection a set-up comprising of laser-induced xenon lamp EQ-99XFC LDLS (Energetiq, USA), a fiber optic multiplexer (Avantes, Netherlands), a flow cell (FIAlab Instruments, USA) and the spectrometer AvaSpec-ULS-TEC with 600 L/mm grating (Avantes, Netherlands) is assembled. This fluorescence device enables excitation light of 7 different wavelengths (Pos. 1-reference, Pos. 2 - 265 nm ± 10 nm, Pos. 3 - 280 nm ± 10 nm, Pos. 4 - 300 nm ± 40 nm, Pos. 5 - 340 nm ± 10 nm, Pos. 6 - 289 nm ± 10 nm, Pos. 7 - 300 nm ± 10 nm, Pos. 8 - 400 nm ± 10 nm) and measurements of whole emission spectra (236 - 795 nm). Additionally, the multi angle light scattering (MALS) detector miniDAWN TREOS (Wyatt, USA) as well as the differential refractive index detector Optilab T-rEX (Wyatt), differential RI in the range of -0.0047 - +0.0047 RIU are applied. All detectors are connected in series, taking into account the peak delay as well as band broadening effects.

Prior to run a chromatography process, the Äkta system and sensors need to be prepared. Äkta pumps are flushed and all lines in the system primed with the respective buffers. The reference cell of the RI detector is filled with running buffer, an ATR-FTIR background spectrum is measured, the MALS flow cell is cleaned with the integrated COMET system and the laser driven fluorescence light source is allowed to warm up for at least 15 minutes. When the preparation work is completed, the chromatography run as well as recording of various signals is started through the EVON control software (evon GmbH). FTIR spectra from 3500 to 750 cm-1 are recorded with a resolution of 4 cm-1. 16 FTIR scans are performed per measurement. Fluorescence spectra are detected over a range of 236 - 795 nm at an integration time of 1 second per excitation wavelength. Light scattering signals are obtained from three integrated detectors at angles of 43.6°, 90° and 136.4°.

The capture step in a chromatography process includes following phases: Column equilibration, loading of the supernatant containing the target protein, washing the column to dispose of weakly bound impurities, followed by elution. When the target protein is eluted, the column is cleaned and re-equilibrated to be ready for the next run.

For training purposes, a certain number of fractions are collected during the elution, which are analyzed subsequently. Concentration, purity and potency are determined in various different analytical methods (e.g. quantity: Reverse Phase-HPLC, Affinity-HPLC, ELISA, potency: surface plasmon resonance assay, cell culture based assay, purity: size exclusion chromatography, LAL endotoxin test, host cell protein ELISA, dsDNA quantification via Picogreen assay). These data are evaluated in context with the obtained online signals. Statistical evaluation and machine learning techniques are applied for predictive model building as described above.

### Offline Methods for measuring quantity, purity and potency used in model building and as control in the Examples

### QUANTITY

### FGF-2 concentration determination

Using a TSK-GEL Superoctyl column for reversed phase HPLC, this methodology allows quantification of human fibroblast growth factor 2 (FGF2). It is based on hydrophobic interactions between the protein and the solid phase of the RP-HPLC column. The FGF2 is eluted at a specific acetonitrile (ACN) concentration. Dual wavelength absorbance is used for detection at 214 and 280 nm.

The method has been developed to determine the total FGF2 concentration. A measurement range from 1.25 µg (0.06 mg/mL) to 30 µg (1.50 mg/mL) with a limit of quantitation of 0.16 µg and a limit of detection of 0.05 µg have been statistically determined.

### mAb concentration determination

Using a monolithic column with protein A as ligand, this methodology allows fast quantification of antibodies. Protein A binds strongly to the Fc region of an antibody and thus can be used for the quantification of whole antibodies (both monoclonal and polyclonal ones) or other macromolecules containing the Fc part of an antibody. The analysis is performed in bind-and-elute mode, where elution is achieved by lowering the pH value with a hydrochloric acid solution. Absorbance is detected at a wavelength of 280 nm.

The method has been developed to determine the total mAb concentration. A measuring range of 0.06 - 0.85 mg/ml has been statistically confirmed and successfully validated.

### PURITY

### Analytical size exclusion chromatography - determination of monomer content and high and low molecular weight impurities

Size exclusion chromatography enables separation of molecules according to their size. This technique can be used to distinguish between the active, monomeric target protein and impurities of higher or lower molecular weight with might be present in the solution.

SEC analysis is performed in isocratic mode, running a protein mixture through a stationary phase which does not interact with the molecules. Bigger molecules cannot enter the pores of the stationary phase, whereas smaller ones can and thus need more time passing through the column. Consequently the molecules are separated by their size and detected by UV absorbance (280 nm and 214 nm respectively).

FGF-2 monomer content is determined using a ACQUITY UPLC BEH125 SEC 1.7µm, 4.6x150mm (Waters) column. The method is run with a buffer comprising 100 mM sodium phosphate, 500 mM NaCl at pH 7.4 at a flow rate of 0.3 ml/min (15 min run). The injection volume is set to 10 µl.

mAb monomer content is determined using a TSKgel G3000 SWxl size exclusion column (Tosoh Bioscience). The method uses 150mM potassium phosphate, pH 6.5 as running buffer at a flow rate of 0.4 ml/min (40 min per run). The injection volume is set 20µl.

### Determination of dsDNA via PicoGreen assay

Quant-iT^{™} PicoGreen^{®} dsDNA reagent is an ultra-sensitive fluorescent nucleic acid stain for quantitating double-stranded DNA (dsDNA) in solution. The linear detection range of the PicoGreen assay is from 1 ng/mL to 1000 ng/mL DNA with a single dye concentration.

The method is based on staining of dsDNA by PicoGreen^{®} dye and fluorometric quantification of the formed conjugate. The standard as well as the sample solutions are diluted in a 96-well plate and subsequently incubated with the colouring reagent for 2 minutes. Signal intensities are then recorded with a plate reader using an excitation of 480 nm and emission filter of 520 nm.

### Determination of host cell protein via ELISA

### E.coli HCP ELISA kit

This kit was developed using broadly reactive polyclonal antibodies to determine the presence of E.coli host cell protein contamination in products manufactured by recombinant expression. Cygnus assay validation has determined that the LOD for this kit is ~0.2ng/mL.

Used antibodies and standard:
- Capture Antibody: Anti-E.coli HCP - , anti-E.coli HCP, af-finity purified (Cygnus AP117)
- Detection Antibody: Anti-E.coli: HRP Conjugate Concentrate; 1mg/mL (Cygnus F411C)
- Standard: E.coli HCP Antigen Concentrate (Cygnus F413H)

### CHO HCP ELISA kit

The CHO ELISA is able to detect HCPs in the range of 100 parts per billion for a variety of antibodies and other therapeutic proteins expressed in CHO.

Used antibodies and standards:
- Capture Antibody: Anti-CHO HCP affinity purified (Cygnus 3G-0016-AF)
- Detection Antibody: Anti-CHO HCP HRP Conjugate Concentrate (Cygnus F551C)
- Standard: CHO HCP Standard (20µg/ml) (Cygnus F553H)

The NUNC MaxiSorp plate is coated with "Anti HCP (affinity purified) antibody". During the first specific reaction step the different HCPs bind to the immobilized "Anti-HCP antibody". During the second reaction step the secondary antibody "Anti- HCP HRP conjugate concentrate" binds to the adsorbed

In the presence of hydrogen peroxide (H2O2) TMB (3,3',5,5'-Tetramethylbenzidine) is oxidized by the horseradish peroxidase (HRP), giving a blue color. After 30 minutes the reaction is stopped by denaturation of the enzyme with 3M sulphuric acid. The color shifts then to yellow and can be measured at 450 nm. The intensity of the staining can be correlated to the amount of bound HCPs.

### POTENCY

### Determination of FGF-2 antigen binding via cell culture assay

### Proliferation assay for bioactivity testing of FGF-2

FGF-2 potency will be measured by an assay using the principle of FGF-2 induced proliferation in NIH-3T3 cells. This fibroblast cell line shows increased proliferation in combination with FGF-2. Cells are seeded in medium with all recommended components (DMEM medium, 10% calf serum, 2mM Na-pyruvate). After 24h calf serum concentration is reduced from 10% to 0.5%. Then cells are treated with increasing concentration of purified FGF-2 (10-100 ng/mL). Commercial FGF-2 is used as positive control and an anti-HER-2 mAb as negative control. For calibration commercial FGF-2 is used (Sinobiological).

### NIH-3T3 seeding in 96-well plate and calf serum reduction

NIH-3T3 cells are cultured in 25 T-flasks in medium containing Dulbecco's Modified Eagle Medium (DMEM), 10 % calf serum, 2mM Na-pyruvate at 37 °C with 5 % CO2, 95 % air and complete humidity. Once the cells reached ~95 % confluency, they are detached using 0.05 % trypsin/EDTA and counted by means of trypan blue in a hemocytometer. These cells are then resuspended at a concentration of 5×105 cells/mL and added onto a 96-well plate (i.e., 200 µL/well resulting in 1.0x105 cells per well) by an 8-channel pipette.

After 24 h 200 µL of DMEM supplemented with only 0.5% calf serum are added to decrease serum concentration in the 96well plates. Serum contains several growth factors. A concentration of 0% calf serum would lead to aggregation of the cells.

### Treating cells with different FGF-2 concentrations

After 36 hours post seeding 20µl or 80µl of the media is exchanged. Then the cultivated cells are treated with selected samples (20µl sample per well). Predilution of samples is done with DMEM medium.

### MTT assay for evaluating of cell viability and proliferation

MTT assay is performed 24 hours after sample addition - 24 h incubation leads to best results in prior experiments. For this purpose, MTT solution is prepared at 1 mg/mL in PBS and is filtered through a 0.2 µm filter. Then, 20 µL of MTT are added to the wells. Cells are incubated for 1 hour at 37 °C with 5 % CO2, 95 % air and complete humidity. After incubation, the MTT containing medium is removed and replaced with 100 µL of DMSO. The plate is further incubated for 30 min at room temperature, and the optical density (OD) of the wells is determined using a plate reader at a test wavelength of 570 nm and a reference wavelength of 690 nm.

### Determination of mAb antigen binding via cell culture assay

### WEHI-164 cytotoxicity assay with rhTNF-α

Anti TNF-α scFv and mAb potency will be measured by an assay using the principle of TNF-α induced apoptosis in WEHI-164 cells. IC50 value of TNF-α to induce apoptosis in WEHI-164 cells has to be determined. Then cells will be treated with IC50 concentration of TNF-α with increasing concentration of purified anti-TNF-α mAb. Commercial anti TNF-α monoclonal antibody will be used as positive control and a nonspecific scFv as negative control. For IC50 determination commercial TNF-α is used (Sinobiological).

### WEHI164 seeding in 96-well plate and Actinomycine D treatment

WEHI164 cells were cultured in 25 T-flasks in medium containing Roswell Park Memorial Institute Medium (RPMI), 10 % FBS at 37 °C with 5 % CO2, 95 % air and complete humidity. Once the cells reached ~95 % confluency, they were detached using 0.05 % trypsin/EDTA and counted by means of trypan blue in a hemocytometer. These cells were then resuspended at a concentration of 7.5×105 cells/mL and added onto a 96-well plate (i.e., 150 µL/well resulting in 1.0x106 cells per well) by an 8-channel pipette. Cell density was optimized in prior experiments.

After 24 h 50 µL of RMPI supplemented with 400 ng/mL Actinomycine D were added to reach a final concentration of 100 ng/mL Actinomycine D in the 96well plates. Optimal concentration of Actinomycine D to increases the sensitivity of WEHI164 to TNF-α was determined in prior experiments - data not shown.

Treating cells with different TNF-α concentrations to determine IC50.

After 36 hours post seeding, media was removed and replaced by 180µl new media supplemented with 100ng/ml Actinomycine D. Then the cultivated cells were treated with selected samples (20µl sample per well). Predilution of samples is done with RPMI medium.

### MTT assay for evaluating cell viability

MTT assay was performed 24 hours after sample addition - 24 h incubation led to best results in prior experiments. For this purpose, MTT solution was prepared at 1 mg/mL in PBS and was filtered through a 0.2 µm filter. Then, 20 µL of MTT was added to the wells. Cells were incubated for 1 hour at 37 °C with 5 % CO2, 95 % air and complete humidity. After incubation, the MTT containing medium was removed and replaced with 100 µL of DMSO. The plate was further incubated for 30 min at room temperature, and the optical density (OD) of the wells was determined using a plate reader at a test wavelength of 570 nm and a reference wavelength of 690 nm.

### Determination of antigen binding via surface plasmon resonance spectroscopy

Surface plasmon resonance (SPR) spectroscopy is a method used for measuring ligand binding interactions. It is a label-free technique to study binding affinities and kinetics of different molecules with an interaction partner, which is immobilized on a sensor surface. It is based on an optical measuring method, which detects small changes in the refractive index on the sensor surface.

With the described method bioactivity of proteins can be determined, such as the following:
- FcyRllla (CD16) - Antibody
- TNF-alpha - antiTNFα antibody
- FGFR2 - FGF-2

Surface plasmon resonance occurs when a polarized light hits a prism covered by a thin (gold) metal layer. At a certain wavelength and incidence angle free electrons at the surface of the biochip absorb the light photons and convert them into surface plasmon waves [1]. Interactions between the immobilized ligand and an analyte at the gold surface of the biochip induce modifications of resonance conditions which are seen as changes in reflectivity and can be measured.

As the refractive index at the interface between the surface and the solution flowing over it changes, the angle of the reflected polarized light alters. The change in angle, caused by binding or dissociation of molecules from the sensor surface, is proportional to the mass of bound material and is recorded in a sensorgram.

The most widely applicable CM5 chip is chosen as the sensor surface combined with amine coupling as the immobilization chemistry. Amine coupling works by forming N-hydroxysuccinimide (NHS) and N-ethyl-N'-(dimethyl-aminopropyl)-carbodiimide hydrochloride (EDC). These esters form covalent links with amine groups on the ligand molecules [2].

0.01 M HEPES, 0.15 M NaCl, 3 mM EDTA, 0.005 % Tween 20, pH 7.4 is used as running buffer in all assays. The specific methods parameters for the respective assay are given in Table 1.

**Table 1 Method parameters for SPR binding assays**

| **Metho d** | **Parameter** | **FcRyllla** | **TNFa** | **FGFR2 / CD332** |
|---|---|---|---|---|
| Immob - ilization | Buffer: | 10 mM Na-acetate, pH 5.5 | 10 mM Na-acetate, pH 5.0 | 10 mM Na-acetate, pH 5.5 |
| | Receptor conc.: | 10 µg/mL | 10 µg/mL | 20 µg/mL |
| | Flow rate: | 5 µL/min | 5 µL/min | 5 µL/min |
| | Immob. target: | approx. 100 RU | approx. 100 RU | approx. 300 RU |
| Kinetics | Flow rate: | 20 µL/min | | |
| | Injection volume: | 12 µL (= 6 min contact time) | | |
| | Dissociation time: | 600 s | 600 s | 500 s |
| | | Samples: | 10 / 25 / 50 / 75 / 100 nM | 1/2/5 /10/20 nM |
| | | Regeneration : | 0.05 % SDS in H₂0 30 µL at flow rate of 30 µL/min (1 min contact time) | 1M MgCl₂x6H₂O 40 µL at flow rate of 20 µL/min (2 min contact time) |

### Examples

The Examples which follow are set forth to aid in the understanding of the invention but are not intended to, and should not be construed to limit the scope of the invention in any way. The Examples do not include detailed descriptions of conventional methods; such methods are well known to those of ordinary skill in the art. General settings and information on offline methods have been summarized above. Schematic overview of tools and principles for a real-time monitored downstream process is shown in Fig. 1.

### Example 1 - Monitoring of an antibody purification by affinity chromatography

CHO-S cells (Invitrogen) were transfected with a combination of 2 plasmids, one carrying the heavy chain gene of trastuzumab and a neomycin resistance gene, the other carrying the light chain of trastuzumab and a DHFR gene with reduced activity. Cells were transfected by electroporation, selected for neomycin resistance and sorted for highest productivity. The resulting pool was subjected to MTX selection (400 nM). Cells were sorted for productivity again, then maintained in culture for 3 months and resorted twice to select for stable producers. During the final sort cells were subcloned by sorting at 1 cell/well. The final clone had a specific productivity of ~20 pg/cell/day.

### Medium and feeds:

Standard medium was CD-CHO (Invitrogen) with 8 mM glutamine and 400 nM MTX. Batch cultures were performed by seeding 2 x 105 cells/ml and cultivation in shaker flasks at 140 rpm, 37 °C and 7 % CO₂. Cultures were terminated when viability dropped below 70 %, typically at day 10. Fed-batch cultures were performed in shake flasks under the same conditions using CHO CD Efficient feed B at 10 % of batch volume at time of feed, plus Function Max Titer Enhancer at 3.3 %.

### Chromatographic process:

A XK column with 16 mm inner diameter (GE Healthcare) was packed with the protein A affinity medium mAb Select SuRe (GE Healthcare) to a total volume of 21.9 ml. The whole process was performed at a flow rate of 75 cm/h. The column was equilibrated over 5 column volumes (CV) with running buffer containing 20 mM Na-phosphate, 150 mM NaCl, pH 7.4. The cell culture supernatant was filtered through a sterile 0.22 µm filter unit (Nalgene) and loaded on the column through the sample pump S9 (GE Healthcare) over 10 CV. Loading was followed by a wash step with 20 mM Na-phosphate, 2 M NaCl, pH 7.4 for 5 CV and another wash step with 20 mM Na-phosphate, 150 mM NaCl, pH 7.4 over 5 CV. Elution of the target protein was achieved with a step gradient over 10 CV with 100 % glycine-HCl at pH 3.5 in equilibration buffer. The eluted fractions were collected in containers holding neutralization buffer with 0.5 M Na-phosphate pH 8.0 (10 % of the fraction volume). The column was sanitized with 0.1 M NaOH over 30 minutes at a low flow rate.

Online sensors (MALS, RI, ATR-FTIR, fluorescence spectroscopy) were prepared for data collection - see "General Settings/ specifications of online sensors for a real-time monitored purification process above".

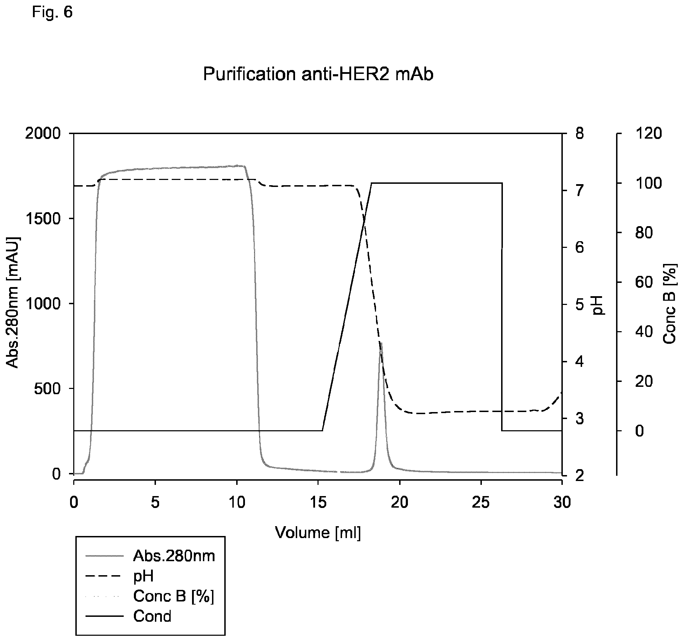

A chromatogram of a capture step of the mAb is presented in Fig. 6.

### Example 2 - Monitoring of purification of basic fibroblast growth factor (FGF-2) from recombinant Escherichia coli

A capture step for the chromatographic purification of basic fibroblast growth factor (FGF-2) from prokaryotic cell culture of recombinant *E. coli* is described and monitored by a battery of online sensors.

The high isoelectric point of FGF-2 (pl 9.4) offers a possibility for an effective removal of a large number of host cell derived impurities by cation exchange chromatography.

Carboxy methyl (CM) sepharose fast flow (weak cation-exchange resin) as a material was used as sorbent in the capture step of FGF-2 from the soluble cell fraction. Eluate fractions of this one-step ion exchange chromatographic procedure were analyzed by offline methods. Offline data are evaluated in context with online signals to predict purity, quantity and potency.

Low cell density (LCD) fermentation of BL21(DE3)_pET30a(cer)_FGF2_ 155(ser78,96). The batch was inoculated with 1 ml of the master cell bank in 30 ml 0.9 % NaCl. The batch cultivation was carried out at 37 °C until a cell dry mass (CDM) of 5.63 g/l was reached. The fed batch cultivation was carried out at 30 °C with an exponential feed rate of µ = 0.1 h⁻¹ for 4 generations to reach a theoretical CDM of 34 g/l. Protein production was induced with 0.9 mM isopropyl β-D-1-thiogalacto-pyranoside (IPTG) per pulse induction after 2 generations. Using this fermentation conditions FGF-2 is produced as soluble protein only and not as inclusion bodies. The cells were harvested 14 h post induction by centrifugation (15 min, 4000 g). The cells were resuspended in homogenization buffer (50 mM TRIS-HCl, 100 mM NaCl, 0.02 % (v/v) TWEEN 20, pH 8.0) to a final concentration of 40 g/l dry cell mass. Cells were disintegrated by passing the cell suspension 2 times through a high pressure homogenisator. Cell debris and insoluble proteins were removed by centrifugation for 30 min and 10,000 g and 4 °C. As supernatant was still turbid after centrifugation step a dead end filtration (0.22 µm) was performed. The clarified supernatant containing soluble FGF-2 was stored at -70 °C.

Online sensors (MALS, RI, ATR-FTIR, fluorescence spectroscopy) were prepared for data collection - see "General Settings/ specifications of online sensors for a real-time monitored purification process above".

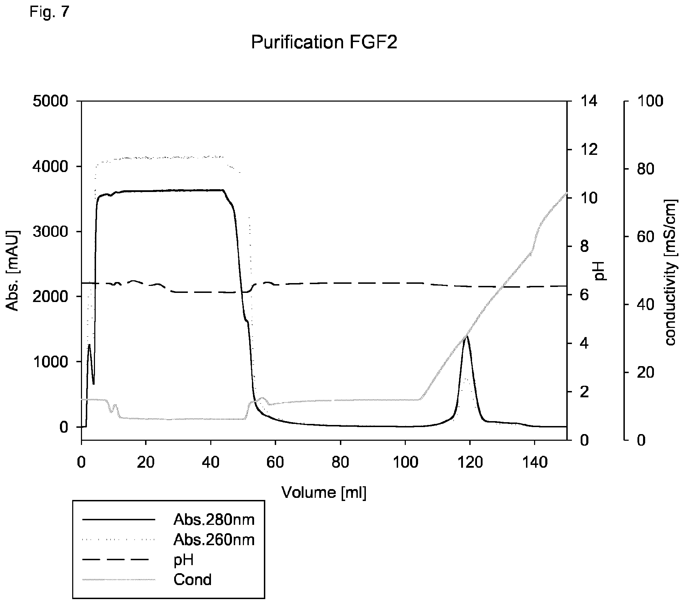

After thawing the supernatant fraction of the cell lysate was applied to a chromatographic column. A laboratory column (Tricorn, i.d. 1 cm, packed column volume 12.3 ml) packed with CM Sepharose^{®} Fast Flow resin (supplier: GE Healthcare) was used employing following method (Table 1). Fig. 7 shows a typical chromatogram of the purification run.

**Table 1: Chromatographic method for FGF-2 purification by application of a CM Sepharose ^{®} Fast Flow resin**

| | | | |
|---|---|---|---|
| Equilibration | 100 mM sodium-phosphate, pH 7.0 | | 5 CV |
| Loading | 0.22 µm filtered supernatant | | 10 CV |
| Wash | 100 mM sodium-phosphate, pH 7.0 | | 5 CV |
| Elution | Elution buffer B: | | |
| | 100 mM sodium-phosphate, 1M NaCl, pH 7.0 | | |
| | | **0-100 % B gradient** | 5 CV |
| | | **100 % B step** | 5CV |
| Re-equilibration | 100 mM sodium-phosphate, pH 7.0 | | 5 CV |
| Flow rate: 150 cm/h | | | |

The sorbents were equilibrated at room temperature with 100 mM Na-phosphate buffer (pH 7.0) using 5 CV. The flow rate was 150 cm/h (1.93 ml/min). Injection was carried out via a sample pump. 10 CV of clarified supernatant (123 ml) were loaded onto the column. After loading the protein solution, the column was washed with 100 mM sodium-phosphate buffer (pH 7.0) with 5 CV until a stable baseline of the UV₂₈₀ signal was reached. FGF-2 was eluted using a linear gradient (5 CV) of 0 - 1 M NaCl in 100 mM sodium-phosphate buffer pH (7.0). Elutes were collected in 1 ml aliquots and offline analyses performed to determine purity (dsDNA content - Picogreen, host cell protein content- *E. coli* HCP ELISA Cygnus), potency (Biacore assay, cell culture bioactivity assay) and quantity (RP-HPLC). The column was treated with 5 CV of 1 M NaCl in 100 mM sodium-phosphate buffer to remove bound impurities. CIP was performed with 10 CV of 0.5 M NaOH solution. Column was re-equilibrated with 100 mM sodium-phosphate buffer (pH 7.0). Long time storage of column is done in 20 % ethanol and 4 °C.

### Example 3 - Monitoring of an Ultradiafiltration (UFDF) of basic fibroblast growth factor - diafiltration and concentration using TFF-ultrafiltration

Ultrafiltration is used either before other process steps to concentrate proteins, for example before chromatography steps, or after purification to reach required concentrations for formulation. Further applications are buffer exchange or removal of impurities. Tangential flow filtration achieves high flux through the membrane by a tangential flow over the membrane, continuously removing the filter cake and preventing clogging of the membrane. The solution is recirculated through the membrane module until the desired concentration is reached, but care has to be taken when designing a process step because of degradation of the membrane performance due to membrane fouling or losing of bioactivity of the target protein due to aggregation. Therefore a set of online sensors (multi angle light scattering, refractive index, fluorescence and ATR-FTIR devices) is implemented after the membrane (retentate stream) of the TFF unit operation.

A lab scale tangential flow filtration unit (Labscale TFF System Millipore) is used for diafiltration and concentrating of FGF-2 eluates after a chromatographic capture step. The lab scale TFF is equipped with pump and pressure sensors. The performance of the membrane in terms of concentration capacity and flux versus time is measured. The effect on the aggregation level of FGF-2 during concentration and the exchange of buffer during diafiltration is monitored by a battery of online sensors. The concentration and diafiltration will be done using a 10 kDa cutoff membrane to ensure that FGF-2 will be retained by the membrane, while buffer passes through.

The purified FGF-2 eluate fractions after the CM-Sepharose Fast Flow chromatography capture step are pooled and diafiltrated against 5 volumes of 50 mM Tris, 150 mM NaCl buffer (pH 7.4). Impurities smaller than 10 kDa cutoff are removed. Finally, an ultrafiltration step is performed to concentrate the retentate 10 fold.

The membrane cassette Kvick start with a cutoff of 10 kDa (GE Healthcare) is connected to the system. The system is started and a transmembrane pressure applied to the membrane, which should not exceed the specifications for the TFF membrane (10 psi). Ultrafiltration with water is performed and the amount of permeate is recorded each minute to determine the normalized water permeability. The system is filled with diafiltration buffer (50 mM Tris, 150 mM NaCl, pH 7.4) and recirculation of buffer is performed. The target protein is filled in the reservoir and a vacuum is applied. Diafiltration is performed to exchange the buffer 5 fold. Due to the vacuum the same amount of buffer is introduced in the system as permeate leaves the system.

Diafiltration is monitored by online sensor (refractive index). The remaining product is concentrated via ultrafiltration manifold. The concentration is monitored via the listed online sensors (multi angle light scattering, fluorescence and ATR-FTIR devices) and concentration process stopped when target concentration is reached or aggregation formation detected. Membrane is flushed with water and 0.5 M NaOH. Long time storage is performed in 20 % ethanol.

A schematic overview of the TFF unit is given in Fig. 8.

### Example 4 - Data Processing

All available data originating from online sensor systems and offline measurements are stored in an EVON database. Selected data sets for a given protein and strain are extracted to comma separated values files and imported into the statistical computing environment R (R Core Team, 2015), where all data processing is performed. As initial steps signal smoothing is conducted for the online signals using the weighted repeated median filter [18] and a background correction of spectral data is performed. Finally, the online data are time aligned with respect to the offline time axes.

For the prediction of protein concentration, purity and potency several machine learning techniques are applied, such as partial least squares regression (PLS [19]), principal component regression (PCR, e.g. [14]), Random Forest (RF [11]), Neural Networks (NN, e.g. [17]) and structured additive regression (STAR, e.g., [13]), which all can deal with a potentially large number of predictors. RF, NN and STAR are especially suited for non-linear processes.

Random Forests runs efficiently on large databases, can handle thousands of input variables without a prior variable selection and gives estimates of which variables are important in the regression problem. RF generates an internal unbiased estimate of the generalization error as the forest building progresses. It is an effective method for estimating missing data, maintains accuracy even if a comparably large proportion of the data is missing and offers an experimental method for detecting variable interactions.

Structured additive regression models can be seen as powerful extensions of linear models allowing for inclusion of e.g. non-parametric smooth effects or interactions between predictors. Boosting as a variable selection tool constructs the final model in a stepwise process by minimizing a loss-function and preserving the additivity of the model structure and provides a variable importance measure via predictor selection frequencies. Generally, the variable selection step is very critical as it needs to provide a relevant subset of inputs for the real time prediction of the responses (protein concentration, purity and potency). Hence, variables that do not provide additional information for the prediction of the response(s) should not be contained in the final model and therefore be removed during variable selection.

From recent studies in the field of upstream processing [15,16] it is known that RF as a variable selection tool and NN as modeling technique as well as STAR models in combination with boosting [12] yield a very good prediction accuracy. All the described algorithms are tested to find the most suitable machine learning technique for the real time monitoring of protein concentration, purity and potency in downstream processes. Method validation is performed on the basis of an independent test set allowing for an estimation of the prediction error in future experiments.

For the establishment of a model based control/release algorithm further information about the process is used and mechanistic models are generated taking into account a priori information such as retention time and the general shape of a chromatogram.

For loading and regeneration model taking into account the capacity for protein or salts, equilibrium models for breakthrough curves, such as equilibrium dispersive model (not mechanistic), pore diffusion model, or combined film and pore diffusion model. For elution same models can be applied, but need the input of the change of conditions with mobile phase modifier.

### Example 5 - Real time monitored FGF-2 purification

A chromatographic purification of basic fibroblast growth factor (FGF-2) from prokaryotic cell culture of recombinant *E. coli* is described and monitored by online sensors (static light scattering, differential refractive index, UV₂₈₀ₙₘ absorbance, ATR-FTIR and fluorescence spectroscopy). Fraction collection is performed based on real time monitored data. Therefore online signals are evaluated with multivariate statistical analysis. A statistical model which is built on an independent training data set is applied to the online signals and used to predict purity, potency and concentration. The system is controlled by software of EVON.

Based on process criteria determined previously (purity > 80 %, potency 100 % and highest possible yield) fractions of this one-step ion exchange chromatographic procedure are collected according predicted process values of online sensors. Purity refers to following definition: 100 % purity is defined as a content of less than 1 ppm (1 ng/mg FGF-2) of dsDNA and less than1 ppm *E. coli* HCP in the sample. Therefore in this example a criteria of 80 % purity means that the impurity content of dsDNA and Host cell protein (HCP) is less than 20 ppm. Potency of 100 % is defined as the bioactivity of the target protein. The bioactive protein has to be is in the right confirmation and not aggregated. Potency is determined via surface plasmon resonance method (Biacore) and cell culture base proliferation assay.

Prior online sensors (MALS, RI, ATR-FTIR, fluorescence spectroscopy) are prepared for data collection- see "General Settings/ specifications of online sensors for a real-time monitored purification process above".

. After thawing the supernatant containing soluble FGF-2 is 0.22 µm filtered. The supernatant fraction is applied to a chromatographic column. A laboratory column (Tricorn, i.d. 1 cm, volume 12.3 ml) packed with CM Sepharose^{®} Fast Flow resin (supplier: GE Healthcare) is used employing the method as described in Table 1.

The sorbents is equilibrated at room temperature with 100 mM Na-phosphate buffer (pH 7.0) using 5 CV. The flow rate is 150 cm/h (1.93 ml/min). Injection is carried out via a sample pump. 10 CV of clarified supernatant (123 ml) is loaded onto the column. After loading the protein solution, the column is washed with 100 mM sodium-phosphate buffer (pH 7.0) with 5 CV until a stable baseline of the UV₂₈₀ signal is reached. FGF-2 is eluted using a linear gradient (5 CV) of 0 - 1 M NaCl in 100 mM sodium-phosphate buffer pH (7.0).

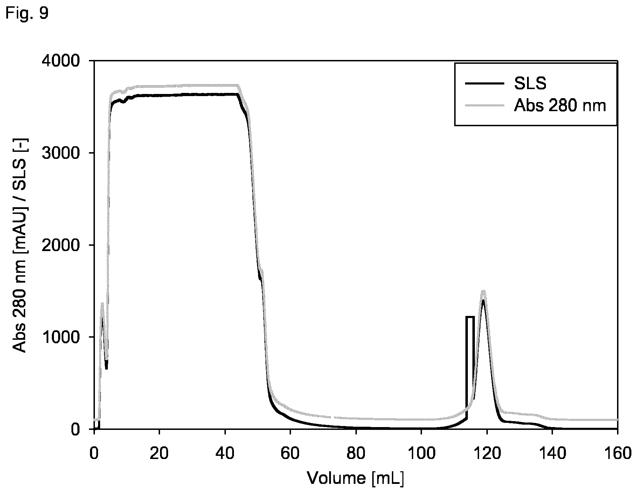

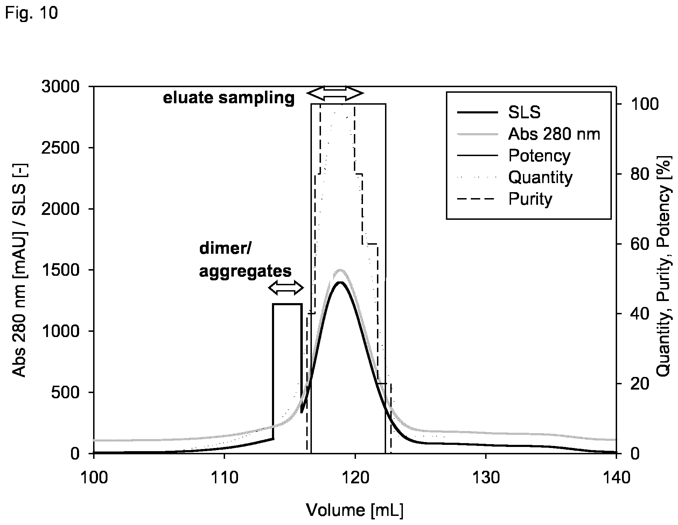

The algorithm starts fraction collection when the process criteria are fulfilled. The absorbance spectrum (UV₂₈₀ₙₘ) is unspecific, as all proteins with aromatic amino acids are detected. The static light scattering signal shows a shoulder at the beginning of the elution peak. This additional signal peak is not detected in the UV₂₈₀ₙₘ signal (Fig. 9). The peak at 115 ml in the SLS chromatogram is due to aggregates and dimer of the target product FGF-2 (Fig. 9). These protein particles have a bigger size. Particles of bigger size lead to stronger scattering of light and thus give information of their physiochemical properties. The potency of the target protein is determined via Biacore method. This offline data are used to train the model for prediction of bioactivity. Protein structure and folding is a major issue for bioactivity. The dimer of FGF-2 is not bioactive and no potency is determined via the algorithm out of the SLS data. The model recognizes that non bioactive dimers of FGF-2 elute from the column (Fig. 10). The change in the fluorescence pattern enables determination of the conformation of the target protein. As the conformation plays a major role in bioactivity these fluorescence data are evaluated in context with offline data. The dimer fraction is not collected for further processing. ATR-FTIR signals are applied to monitor host cell protein content and therefore the purity of the eluate (Fig. 10). Proteins have a unique AFT-FTIR spectrum in the amide region referred as fingerprint region (1500 - 500 cm⁻¹). The change of the ATR-FTIR pattern enables to distinguish between host cell protein and target protein. The start and the end of the elution peak contain major host cell impurities. At the beginning weak bound impurities are removed wherein at the elution tail strongly bound impurities are removed by the salt gradient. A purity of at least 80 % is defined as collection criteria for further processing. Target protein content of target protein FGF-2 is quantified via UV₂₈₀ₙₘ and ATR-FTIR signal. The quantity is determined in two orthogonal methods, whereas ATR-FTIR is the more specific. UV₂₈₀ₙₘ quantifies aromatic amino acids, therefore also host cell proteins are co-quantified.

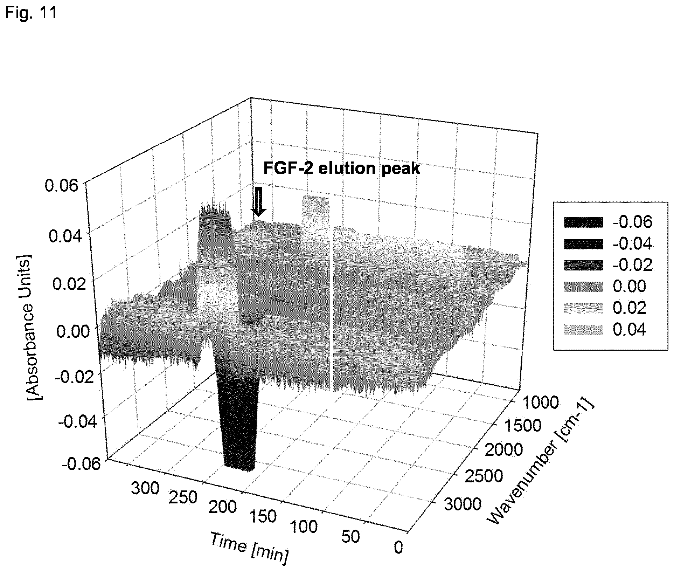

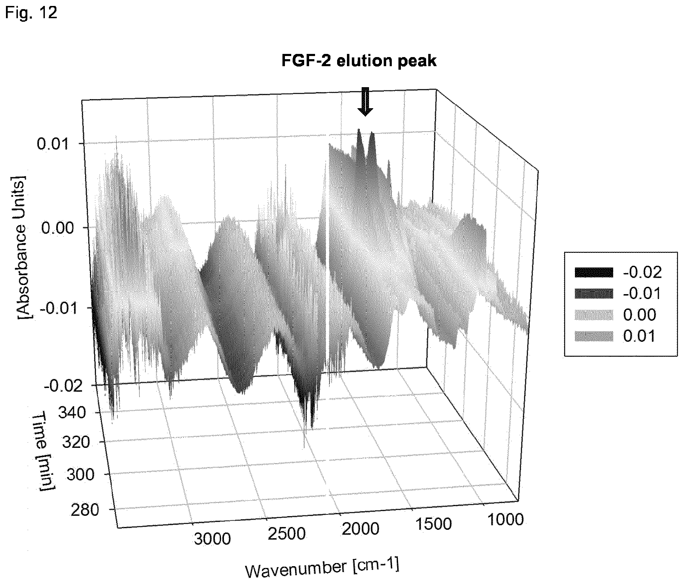

Different method blocks can be clearly differentiated in the ATR-FTIR spectrum - as shown in Fig. 11 and Fig. 12. During the loading elevated signals can be seen at the range of 1500 - 1600 cm⁻¹ which is known as the amide II band. This is caused by the high amount of host cell protein that is contained in the supernatant. The wash block is indicated by the very striking bands at the region > 3000 cm⁻¹. But also the elution block shows a distinct profile referred to the target protein FGF-2. The eluting protein causes a peak at 1528 cm⁻¹. A zoom of the ATR-FTIR signal caused by the elution phase can be seen in Fig. 12. Again in the range between 1500 and 1600 cm⁻¹ the gradient is also visible in the ATR-FTIR spectrum. Not only this part of the spectrum contains valuable information for the modelling. The peak is evaluated in context with offline quantity data to determine concentration and host cell protein content.

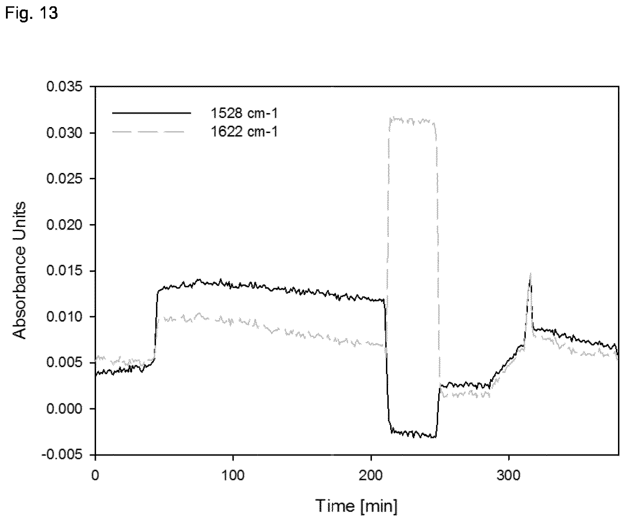

Fig. 13 illustrates two wavenumbers (1528 cm⁻¹ and 1622 cm⁻¹) of the amide bands over the course of the chromatography run, where again the different method block can be distinguished. The equilibration buffer is only pronounced at 1622 cm⁻¹, whereas the elution peak looks similar in both lines.

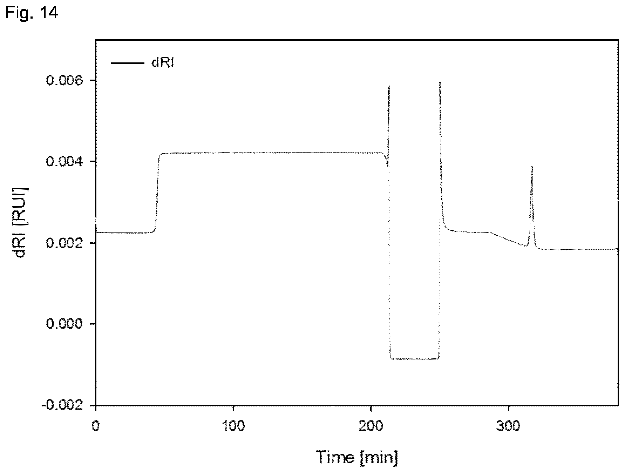

The signal of the refractive index detector is displayed in Fig. 14. Again the individual method blocks can clearly be differentiated. In the elution peak the additional contribution to the RI signal of the eluting target protein can be detected. Intrinsic fluorescence of proteins is mainly caused by aromatic amino acids (tryptophan and tyrosine). Highest emission occurs at around 350 nm at an excitation wavelength of 295 nm. Based on the change and shape of the fluorescence pattern the bioactivity the eluting protein can be determined.

### Example 6 - Real time monitoring to determine breakthrough of a loading step of FGF-2 on a Heparin Sepharose Fast flow column

A chromatographic purification of basic fibroblast growth factor (FGF-2) from prokaryotic cell culture of recombinant *E. coli* is described and monitored by online sensors (static light scattering, differential refractive index, UV₂₈₀ₙₘ absorbance, ATR-FTIR and fluorescence spectroscopy). Loading step on column is performed based on real time monitored data. Online signals are evaluated by means of multivariate statistical analyses. A statistical model which is built on an independent training data set is applied to the online signals and used to predict breakthrough of target protein. The system is controlled by software of EVON.

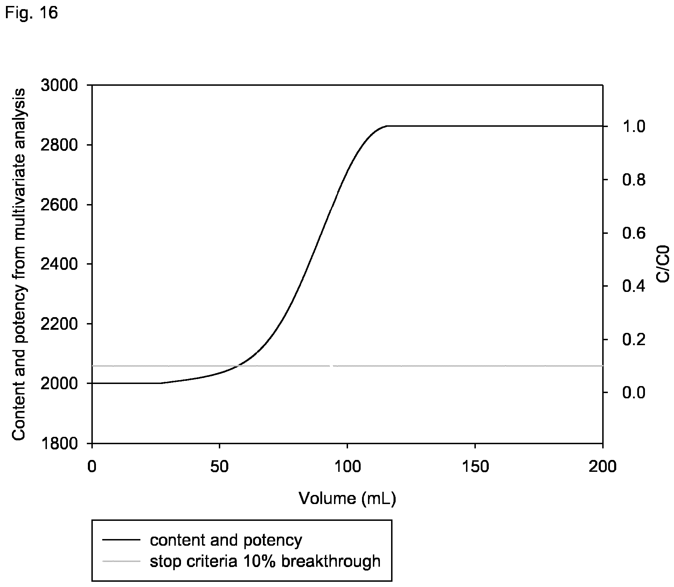

Process stop criteria are defined: FGF-2 concentration in flow through: C/C₀ < 10 %) - see Fig. 16. Where C₀ is the concentration of FGF-2 in the supernatant feed and C the concentration of FGF-2 in the flow through. Online sensors are applied to determine the increase of target protein in the flow through. When the capacity of the column is reached the loading process is stopped via EVON software. The algorithm stops loading when the process criteria are fulfilled. The absorbance spectrum (UV₂₈₀ₙₘ) is unspecific, as all proteins with aromatic amino acids are detected. A combination of UV, ATR-FTIR, MALS, RI and fluorescence device enables to distinguish between HCP and target protein. Especially ATR-FTIR allows distinguishing between host cell proteins and target protein FGF-2. Signal pattern and shifts provides information if composition of flow through changes. If the target protein reaches a certain concentration in flow through the washing step is initiated via control software and the purification process is continued. Controlling loading conditions of the resin by this means enables utilization of binding capacities in an economical way.

Online sensors (MALS, RI, ATR-FTIR, fluorescence spectroscopy) are prepared for data collection. After thawing the supernatant containing soluble FGF-2 is 0.22 µm filtered. The supernatant fraction is applied to a chromatographic column. A laboratory column (Tricorn, i.d. 1 cm, volume 12.0 ml) packed with Heparin Sepharose^{®} Fast Flow resin (supplier: GE Healthcare) is used.

The sorbents is equilibrated at room temperature with 20 mM Tris-HCl buffer, 400 mM NaCl (pH 7.4) using 5 CV. The flow rate is 150 cm/h (1.93 ml/min). Injection is carried out via a sample pump. Clarified supernatant is loaded onto the column as long as breakthrough criteria are not fulfilled. After loading the protein solution, the column is washed with 20 mM Tris-HCl buffer, 400 mM NaCl (pH 7.4) with 5 CV until a stable baseline of the UV₂₈₀ₙₘ signal is reached. FGF-2 is eluted using a linear gradient (5 CV) of 0.4 - 2 M NaCl in 20 mM sodium-phosphate buffer pH (7.4).

### Example 7 - Data Preprocessing, Statistical Modeling and Validation in one run

Online data available for modeling comprise signals from the UV-VIS, Conductivity, Pressure, pH, MALS and RI devices (with p = 14 variables in total), ATR-FTIR spectra (resolution approximately 2 cm⁻¹ resulting in p = 1427 predictors) as well as fluorescence emission spectra at 7 excitation wavelengths (resolution about 0.3 nm) giving in total 14366 fluorescence variables. Depending on the predictor sets and the response variable (Quantity which means the same as Concentration), Host Cell Protein (HCP), double-stranded DNA (dsDNA), Monomer and High Molecular Weight HMW impurity concentrations or the Potency expressed as the KD value of the receptor biological product interaction), data from 7 to 14 chromatographic runs are available for model building.

Infrared and fluorescence spectra are smoothed using the Savitzky-Golay filter, the former are also baseline corrected using 2^{nd} derivative constrained weighted regression as implemented in the R packages signal and baseline.

Finally, a time alignment step is performed - averages are calculated for each online variable corresponding to the time frame of each offline fraction considering the known time delay between several devices.

Results shown in this section are obtained by STAR (structured additive regression) models in combination with boosting as a variable selection technique (R package *mboost*). Parameter optimization and model selection is performed on autoscaled data (i.e. from each predictor the mean is subtracted and divided by its standard deviation) via cross validation (data from each run are left out once and predicted by a model based on the data of several other runs). The model quality is measured with the cross-validated root-mean-squared error (RMSE).

Exemplary modeling results and a comparison to the corresponding offline values for the subsequent target variables are shown:
1. Protein quantity (concentration) in mg/ml (Fig. 17)
2. Host cell protein concentration in ng/ml and relative to the estimated protein quantity in ppm (Fig. 18 and 19)
3. Double-stranded DNA (dsDNA) concentration in ng/ml and relative to the estimated protein quantity in ppm (Figs. 20 and 21)
4. Monomer and high molecular weight impurities in % (Figs. 22 and 23)
5. FGF2 Potency expressed by the KD value of the receptor FGF2 interaction (Fig. 24)

Corresponding results are presented in figures 17 (Quantity), 18-19 (Host Cell Protein in ng/ml and ppm, respectively), 20-21 (dsDNA in ng/ml and ppm), 22-23 (monomer and high molecular weight impurities fractions) and 24 (potency KD value). In these figures, actually measured offline values for the targets are given as bars, the model predictions based on online data on a time grid of 1 second are depicted as curves and these predictions averaged over each offline fraction are shown as horizontal lines.

Results are given for a single chromatographic run, with prediction errors similar to the overall prediction error (averaged over all runs). The horizontal axis represents time (in *minutes*) with the origin placed at the start of the first offline fraction.
a) **Protein Quantity:** Overall prediction error of RMSE = 0.44 mg/ml (average for all runs) - as an example the results from run 3 show a performance of RMSE = 0.25 mg/ml (figure 17); the model is based on predictors of the UV-VIS, pressure, conductivity, pH, MALS and RI devices and uses data of 14 runs, which are predicted with accuracies between 0.23 and 0.86 mg/ml; the STAR model uses an intercept, linear and non-parametric smooth baselearners.
b) **Host Cell Protein (HCP):** Average prediction error of RMSE = 46.4 ng/ml (run 3 given as an example exhibits an error of about 43 ng/ml, figure 18). The model is based on UV-VIS, pressure, conductivity, MALS, RI and fluorescence signals (the latter at excitation wavelengths of 280 and 400 nm). Besides intercept, linear and smooth predictor functions product interactions between the UV-VIS, pressure, conductivity, pH, MALS and RI predictors are used. Within the cross-validation, runs are predicted with errors between 24.0 and 63.2 ng/ml.
   Both the HCP and quantity prediction models can also be used to predict the host cell protein in units of ng per mg protein (ppm), as shown in figure 19.
c) **Double-stranded DNA (dsDNA):** Results for this target are shown in figures 20 (in units ng/ml) and 21 (in ppm, i.e. relative to the estimated protein quantity). Runs are predicted with an average error of 77.2 ng/ml. Single runs exhibit cross-validated prediction errors between about 41 and 99 ng/ml. The model considered here uses predictors from the pH, MALS, RI and Fluorescence devices. Similar to previous models, intercept, linear, smooth baselearners and their product interactions are used for the pH, MALS and RI variables, whereas only smooth functions of the fluorescence predictors enter the model.
d) **Protein Monomer and High Molecular Weight Impurities (HMW):** Figures 22 and 23 show the results for modeling the monomer and high molecular weight impurities fractions (both as fractions of one, i.e. their values add up to 1) for run 3. The model uses information of the UV-VIS, pH, Conductivity, MALS, RI and Fluorescence devices and yields an average performance of RMSE = 0.056 (with values ranging from 0.025 to 0.08 for the various runs). Baselearners of the same functional types as for the dsDNA models are used in this case.
e) **Potency (KD value):** the average prediction error for the *potency* response (measured via its KD value) is RMSE = 0.97 (the prediction performance determined for run 3 shown in figure 24 is RMSE = 1.38). Predictors from several devices (UV-VIS, Conductivity, Pressure, pH, MALS, RI, Fluorescence and ATR-FTIR) are used. Besides linear functions of several predictors smooth functions of UV-VIS, Conductivity, Pressure, pH, MALS, RI and Fluorescence signals as well as product interactions between the UV-VIS, Conductivity, Pressure, pH, MALS and RI signals are incorporated in the prediction model.

### References

[1] M.M.C. Van Beers, M. Bardor, Minimizing immunogenicity of biopharmaceuticals by controlling critical quality attributes of proteins, Biotechnology Journal, 7 (2012) 1473-1484.
[2] F. Capito, R. Skudas, Applications and limitations of FT-MIR for monitoring critical process parameters during downstream processing of therapeutic proteins, in: Infrared Spectroscopy: Theory, Developments and Applications, 2014, pp. 205-246.
[3] F. Capito, R. Skudas, H. Kolmar, C. Hunzinger, At-line mid infrared spectroscopy for monitoring downstream processing unit operations, Process Biochemistry, 50 (2015) 997-1005.
[4] F. Capito, A. Zimmer, R. Skudas, Mid-infrared spectroscopy-based analysis of mammalian cell culture Parameters, Biotechnology Progress, 31 (2015) 578-584.
[5] I. Strug, C. Utzat, A. Cappione, S. Gutierrez, R. Amara, J. Lento, F. Capito, R. Skudas, E. Chernokalskaya, T. Nadler, Development of a univariate membrane-based mid-infrared method for protein quantitation and total lipid content analysis of biological samples, Journal of Analytical Methods in Chemistry, 2014 (2014).
[6] F. Capito, R. Skudas, H. Kolmar, B. Stanislawski, Host cell protein quantification by fourier transform mid infrared spectroscopy (FT-MIR), Biotechnology and Bioengineering, 110 (2013) 252-259.
[7] F. Capito, R. Skudas, B. Stanislawski, H. Kolmar, Matrix effects during monitoring of antibody and host cell proteins using attenuated total reflection spectroscopy, Biotechnology Progress, 29 (2013) 265-274.
[8] L. Dagge, K. Harr, M. Paul, G. Schnedl, Classification of process analysis: Offline, atline, online, inline, Cement International, 7 (2009) 72-81.
[9] R.H. Clemmitt, L.J. Bruce, H.A. Chase, On-line monitoring of the purification of GST-(His)6 from an unclarified Escherichia coli homogenate within an immobilised metal affinity expanded bed, Bioseparation, 8 (1999) 53-67.
[10] Klockewitz, K., Riechel, P., Hagedorn, J., Scheper, T., Noe, W., Howaldt, M., Vorlop, J. Fast FIA-immunoanalysis systems for the monitoring of downstream processes (2000) Chemical and Biochemical Engineering Quarterly, 14 (2), pp. 43-46.
[11] Breiman, L. (2001). Random forests. Mach. Learn., 45, 5-32.
[12] Bühlmann, P., Hothorn, T. (2007). Boosting Algorithms: Regularization, Prediction and Model Fitting. Statistical Science 22:477-505.
[13] Fahrmeir, L., Kneib, T., Lang, S. (2009). Regression - Modelle, Methoden und Anwendungen. Heidelberg Dordrecht London New York: Springer. 502 p.
[14] Hastie, T., Tibshirani, R., Friedman, J. (2009). The Elements of Statistical Learning: Prediction, Inference and Data Mining, Second Edition, Springer Verlag.
[15] Melcher, M., Scharl, T., Spangl, B., Luchner, M., Cserjan, M., Bayer, K., Leisch, F., Striedner, G. (2015). The potential of random forest and neural networks for biomass and recombinant protein modeling in Escherichia coli fed-batch fermentations. Biotechnol J 10:1770-1782.
[16] Melcher, M., Scharl, T., Luchner, M., Striedner, G., Leisch, F. (2016). Boosted structured additive regression models in Escherichia coli fed-batch fermentation modeling. In Preparation.
[17] Ripley, B. D. (1996). Pattern Recognition and Neural Networks, University Press, Cambridge.
[18] Schettlinger, K., Fried, R., Gather, U. (2010). Real Time Signal Processing by Adaptive Repeated Median Filters, International Journal of Adaptive Control and Signal Processing, 2010, 24(5), 346-362.
[19] Wold, S., Sjöström, M., Eriksson, L. (2001). PLS-regression: A basic tool of chemometric. Chemom. Intell. Lab. Syst., 58, 109-130.
[20] Software: R Core Team (2015). R: A language and environment for statistical computing. R Foundation for Statistical Computing, Vienna, Austria. URL http://www.R-project.org/

## Claims

1. A computer-based method for monitoring the purification and/or concentration process of a biological product which comprises the use of at least one operation unit, wherein the method comprises the steps of
a. including at least two independent online sensors in the operation unit,
b. obtaining a plurality of data values corresponding to the respective output of said online sensors;
c. importing the data values into a computer database, where they are evaluated by a predictive multivariate statistical analysis model obtained previously from online data, which were obtained from said online sensors, and from offline data, which were obtained based on samples being removed from the process, thereby performing a multivariate statistical analysis for the prediction of concentration, purity and/or potency of the biological product,
d. diagnosing the actual data values corresponding to the respective output of said multivariate statistical analysis;
e. monitoring the concentration, purity and/or potency of the biological product in real time.

2. The method according to claim 1, wherein at least one of the online sensors is selected from the group consisting of multi-angle light scattering sensors (MALS), UV-VIS absorption sensors, fluorescence sensors, infrared absorption sensors (IR), attenuated total reflection-fourier transform infrared spectroscopy sensors (ATR-FTIR), refractive index (RI) sensors, pH sensors, temperature sensors, conductivity sensors, pressure sensors, small angle x-ray scattering (SAXS) sensors and redox sensors.

3. The method according to claim 1 or 2, wherein at least one of the online sensors is selected from the group consisting of ATR-FTIR, MALS, RI and fluorescence sensors.

4. The method according to any one of claims 1-3, wherein at least one of the online sensors is a non-invasive *in situ* sensor;
wherein the non-invasive *in-situ* sensor is selected from the group consisting of ATR-FTIR, SAXS, temperature, pH, conductivity and redox sensors

5. The method according to any one of claims 1-4, wherein the method further comprises the steps of:
f. based on the information obtained in e) performing process regulation and/or process optimization, and
g. optionally based on the information obtained in e) parametric or real time release of the biological product.

6. The method according to any one of claims 1-5, wherein the operation unit comprises at least three, at least four, at least five, at least 6, at least 7, at least 8, at least 9, at least 10 or more independent online sensors.

7. The method according to any one of claims 1-6, wherein the operation unit comprises at least one ATR-FTIR sensor, one MALS sensor, one RI sensor and one fluorescence sensor.

8. The method according to claim 7, wherein the operation unit additionally comprises at least one temperature sensor, at least one conductivity sensor, at least one pH sensor and at least one pressure sensor.

9. The method according to claim 7 or 8, wherein at least one of the sensors is a non-invasive *in-situ* sensor selected from the group consisting of ATR-FTIR sensors, temperature sensors, SAXS sensor, conductivity sensors, pH sensors and pressure sensors.

10. The method according to any one of claims 1-9, wherein the operation unit comprises a chromatography unit and/or filtration unit.

11. The method according to claim 10, wherein the chromatography unit is selected from the group consisting of ion exchange chromatography, affinity chromatography, size exclusion chromatography, reversed phase chromatography, hydrophobic interaction chromatography, multi-modal resin chromatography, operated in isocratic, linear, segmented and/ or step gradient elution in bind/elute or flow through mode and the filtration unit is selected from the group consisting of ultrafiltration, microfiltration, nanofiltration, depth filtration, operated in tangential flow filtration, dead end filtration, filtration through absolute pore size membranes.

12. The method according to any one of claims 5-11, wherein in step f) the process is regulated with regard to any one or a combination of peak collection, correct collection of the biological product after refolding, filtration or precipitation, product quality, and process equipment maintenance.

13. The method according to any one of claims 1-12, wherein said biological product is a biopharmaceutical, a nucleic acid molecule or heterologous protein, preferably selected from therapeutic proteins, enzymes and peptides, protein antibiotics, fusion proteins, carbohydrate - protein conjugates, structural proteins, regulatory proteins, vaccines and vaccine like proteins or particles, process enzymes, growth factors, hormones and cytokines, antibodies or a metabolite of said biological product.

14. A method for producing a biological product comprising the steps of
a. culturing an organism or a cell capable of producing the biological product;
b. purifying the product resulting from step a), wherein purification is monitored according to any one of claims 1-13.

15. A device comprising an operation unit for purification and/or concentration of a biological product, wherein the operation unit comprises
a chromatography and/or a filtration unit,
wherein the chromatography or filtration unit comprises at least one online sensor, and
wherein the sensor is connected to a computer, where the data values collected by said sensor are imported into a database and where they are evaluated by a predictive multivariate statistical analysis model obtained previously from online data which were obtained from said online sensor, and from offline data which were obtained based on samples being removed from the process thereby performing a multivariate statistical analysis for the prediction of concentration, purity and/or potency of said biological product and where the actual data values are then diagnosed corresponding to the respective output of said multivariate statistical anal.

16. The device according to claim 15, wherein the at least one online sensor is selected from the group consisting of multi-angle light scattering sensors (MALS), UV-VIS absorption sensors, fluorescence sensors, infrared absorption sensors (IR), attenuated total reflection-fourier transform infrared spectroscopy sensors (ATR-FTIR), light refractive index (RI) sensors, pH sensors, temperature sensors, conductivity sensors, pressure sensors, small angle x-ray scattering (SAXS) sensors and redox sensors.

## Patentansprüche

1. Computer-implementiertes Verfahren zur Überwachung des Reinigungs- und/oder Konzentrationsprozesses eines biologischen Produkts, das die Verwendung von mindestens einer Betriebseinheit umfasst, wobei das Verfahren die Schritte aufweist:
a. Bereitstellen von mindestens zwei unabhängigen Online-Sensoren in der Betriebseinheit,
b. Erlangen einer Vielzahl von Datenwerten, die der jeweiligen Ausgabe der Online-Sensoren entsprechen;
c. Importieren der Datenwerte in eine Computerdatenbank, wo sie durch ein prädiktives multivariates statistisches Analysemodell ausgewertet werden, das zuvor aus Online-Daten gewonnen wurde, die von den Online-Sensoren gewonnen wurden, und aus Offline-Daten, die auf Basis von Proben gewonnen wurden, die aus dem Prozess entnommen wurden, wodurch eine multivariate statistische Analyse durchgeführt wird zur Vorhersage der Konzentration, Reinheit und/oder Wirksamkeit des biologischen Produkts,
d. Diagnostizieren der tatsächlichen Datenwerte, die der jeweiligen Ausgabe der multivariaten statistischen Analyse entsprechen;
e. Überwachen der Konzentration, Reinheit und/oder Wirksamkeit des biologischen Produkts in Echtzeit.

2. Verfahren nach Anspruch 1, wobei mindestens einer der Online-Sensoren aus der Gruppe bestehend aus Mehrwinkel-Lichtstreusensoren (MALS), UV-VIS-Absorptionssensoren, Fluoreszenzsensoren, Infrarot-Absorptionssensoren (IR), abgeschwächten Totalreflexions-Fourier-Transformations-Infrarotspektroskopie-Sensoren (ATR-FTIR), Brechungsindexsensoren (RI), pH-Sensoren, Temperatursensoren, Leitfähigkeitssensoren, Drucksensoren, Kleinwinkel-Röntgenstreusensoren (SAXS) und Redoxsensoren ausgewählt ist.

3. Verfahren nach Anspruch 1 oder 2, wobei mindestens einer der Online-Sensoren aus der Gruppe bestehend aus ATR-FTIR, MALS, RI und Fluoreszenzsensoren ausgewählt ist.

4. Verfahren nach einem der Ansprüche 1 bis 3, wobei mindestens einer der Online-Sensoren ein nicht-invasiver in-situ-Sensor ist;
wobei die nicht-invasive in-situ Sensor aus der Gruppe bestehend aus ATR-FTIR, SAXS, Temperatur-, pH-, Leitfähigkeits- und Redoxsensoren ausgewählt ist.

5. Verfahren nach einem der Ansprüche 1 bis 4, wobei das Verfahren ferner die Schritte umfasst:
f. auf der Basis der in e) erhaltenen Informationen, Durchführen einer Prozessregelung und/oder Prozessoptimierung, und
g. optional, auf Basis der in e) erhaltenen Informationen, parametrisches oder Echtzeit-Freigeben des biologischen Produkts.

6. Verfahren nach einem der Ansprüche 1 bis 5, wobei die Betriebseinheit mindestens drei, mindestens vier, mindestens fünf, mindestens sechs, mindestens sieben, mindestens acht, mindestens neun, mindestens zehn oder mehr unabhängige Online-Sensoren umfasst.

7. Verfahren nach einem der Ansprüche 1 bis 6, wobei die Betriebseinheit mindestens einen ATR-FTIR-Sensor, einen MALS-Sensor, einen RI-Sensor und einen Fluoreszenzsensor umfasst.

8. Verfahren nach Anspruch 7, wobei die Betriebseinheit zusätzlich mindestens einen Temperatursensor, mindestens einen Leitfähigkeitssensor, mindestens einen pH-Sensor und mindestens einen Drucksensor umfasst.

9. Verfahren nach Anspruch 7 oder 8, wobei mindestens einer der Sensoren ein nicht-invasiver in-situ-Sensor ist, ausgewählt aus der Gruppe bestehend aus ATR-FTIR-Sensoren, Temperatursensoren, SAXS-Sensoren, Leitfähigkeitssensoren, pH-Sensoren und Drucksensoren.

10. Verfahren nach einem der Ansprüche 1 bis 9, wobei die Betriebseinheit eine Chromatographieeinheit und/oder eine Filtrationseinheit umfasst.

11. Verfahren nach Anspruch 10, wobei die Chromatographieeinheit ausgewählt ist aus der Gruppe bestehend aus einer Ionenaustauschchromatographie, einer Affinitätschromatographie, einer Größenausschlusschromatographie, einer Umkehrphasenchromatographie, einer hydrophoben Interaktionschromatographie, multimodaler Harzchromatographie, betrieben in isokratischer, linearer, segmentierter und/oder Stufengradientenelution im Bind/Elue- oder Flow-Through-Modus, und die Filtrationseinheit ausgewählt ist aus der Gruppe bestehend aus Ultrafiltration, Mikrofiltration, Nanofiltration, Tiefenfiltration, betrieben in der Tangentialfiltration, Dead-End-Filtration, Filtration durch Membranen mit absoluter Porengröße.

12. Verfahren nach einem der Ansprüche 5 bis 11, wobei in Schritt f) das Verfahren in Bezug auf eine beliebige oder eine Kombination von Spitzensammlung, korrekter Sammlung des biologischen Produkts nach der Rückfaltung, Filtration oder Fällung, Produktqualität und Wartung der Prozessausrüstung geregelt ist.

13. Verfahren nach einem der Ansprüche 1 bis 12, wobei das biologische Produkt ein Biopharmazeutikum, ein Nukleinsäuremolekül oder ein heterologes Protein ist, vorzugsweise ausgewählt aus therapeutischen Proteinen, Enzymen und Peptiden, Proteinantibiotika, Fusionsproteinen, Kohlenhydrat-Protein-Konjugaten, Strukturproteinen, regulatorischen Proteinen, Impfstoffen und impfstoffähnlichen Proteinen oder Partikeln, Prozessenzymen, Wachstumsfaktoren, Hormonen und Zytokinen, Antikörper oder einen Metaboliten des biologischen Produkts ist.

14. Verfahren zur Herstellung eines biologischen Produkts, umfassend die Schritte
a. Kultivieren eines Organismus oder einer Zelle, die in der Lage ist, das biologische Produkt zu produzieren;
b) Reinigen des Produkts, das sich aus Schritt a) ergibt, wobei die Reinigung gemäß einem der Ansprüche 1 bis 13 überwacht wird.

15. Vorrichtung, aufweisend eine Betriebseinheit zur Reinigung und/oder Konzentration eines biologischen Produkts, wobei die Betriebseinheit aufweist:
eine Chromatographie- und/oder eine Filtrationseinheit, wobei die Chromatographie- oder Filtrationseinheit mindestens einen Online-Sensor aufweist, und
wobei der Sensor mit einem Computer verbunden ist, wobei die von dem Sensor gesammelten Datenwerte in eine Datenbank importiert werden und wo sie durch ein prädiktives multivariates statistisches Analysemodell ausgewertet werden, das zuvor aus Online-Daten, die von dem Online-Sensor erhalten wurden, und aus Offline-Daten erhalten wurde,die auf Basis von Proben erhalten wurden, die aus dem Prozess entnommen wurden, wodurch eine multivariate statistische Analyse zur Vorhersage der Konzentration, Reinheit und/oder Wirksamkeit des biologischen Produkts durchgeführt wurde, und wo die tatsächlichen Datenwerte dann entsprechend der jeweiligen Ausgabe der multivariaten statistischen Analyse diagnostiziert werden.

16. Vorrichtung nach Anspruch 15, wobei der mindestens eine Online-Sensor aus der Gruppe bestehend aus Mehrwinkel-Lichtstreusensoren (MALS), UV-VIS-Absorptionssensoren, Fluoreszenzsensoren, Infrarot-Absorptionssensoren (IR), abgeschwächten Totalreflexions-Fourier-Transformations-Infrarotspektroskopie-Sensoren (ATR-FTIR), Lichtbrechungsindex-Sensoren (RI), pH-Sensoren, Temperatursensoren, Leitfähigkeitssensoren, Drucksensoren, Kleinwinkel-Röntgenstreusensoren (SAXS) und Redoxsensoren ausgewählt ist.

## Revendications

1. Méthode mise en œuvre par ordinateur pour surveiller un processus de purification et/ou de concentration d'un produit biologique qui comprend l'utilisation d'au moins une unité d'opération, dans lequel le procédé comprend les étapes :
a. fournir au moins deux capteurs en ligne indépendants,
b. obtenir d'une pluralité de valeurs de données correspondant à la sortie respective desdits capteurs en ligne ;
c. importer des valeurs de données dans une base de données informatique, où elles sont évaluées par un modèle d'analyse statistique multivarié prédictif obtenu précédemment à partir de données en ligne , obtenues à partir desdits capteurs en ligne, et à partir de données hors ligne, obtenues sur la base d'échantillons retirés du processus, effectuant ainsi une analyse statistique multivariée pour la prédiction de la concentration, de la pureté et/ou de l'activité du produit biologique,
d. diagnostiquer les valeurs réelles des données correspondant aux résultats respectifs de ladite analyse statistique multivariée ;
e. surveiller la concentration, la pureté et/ou l'activité du produit biologique en temps réel.

2. Méthode selon la revendication 1, dans lequel au moins un des capteurs en ligne est sélectionné dans le groupe constitué de capteurs de diffusion de lumière multi-angles (MALS), de capteurs d'absorption UV-VIS, de capteurs de fluorescence, de capteurs d'absorption infrarouge (IR), de capteurs de spectroscopie infrarouge à transformée de Fourier à réflexion totale atténuée (ATR-FTIR), de capteurs d'indice de réfraction (IR), de capteurs de pH, de capteurs de température, de capteurs de conductivité, de capteurs de pression, de capteurs de diffusion des rayons X aux petits angles (SAXS) et de redox capteurs.

3. Méthode selon la revendication 1 ou 2, dans lequel au moins un des capteurs en ligne est sélectionné dans le groupe constitué par ATR-FTIR, MALS, RI et capteurs de fluorescence.

4. Méthode selon l'une quelconque des revendications 1 à 3, dans lequel au moins un des capteurs en ligne est un capteur in situ non invasif;
dans lequel le in-situ le capteur est choisi dans le groupe composé des capteurs ATR-FTIR, SAXS, de température, de pH, de conductivité et d'oxydoréduction.

5. Méthode selon l'une quelconque des revendications 1 à 4, dans lequel la Méthode comprend en outre les étapes consistant à:
f. sur la base des informations obtenues dans e) la régulation et/ou l'optimisation du processus, et
g. éventuellement sur la base des informations obtenues dans e) la libération paramétrique ou en temps réel du produit biologique.

6. Méthode selon l'une quelconque des revendications 1 à 5, dans lequel l'unité de fonctionnement comprend au moins trois, au moins quatre, au moins cinq, au moins 6, au moins 7, au moins 8, au moins 9, au moins 10 capteurs en ligne indépendants ou plus.

7. Méthode selon l'une quelconque des revendications 1 à 6, dans lequel l'unité de fonctionnement comprend au moins un capteur ATR-FTIR, un capteur MALS, un capteur RI et un capteur de fluorescence.

8. Méthode selon la revendication 7, dans lequel l'unité de commande comprend en outre au moins un capteur de température, au moins un capteur de conductivité, au moins un capteur de pH et au moins un capteur de pression.

9. Méthode selon la revendication 7 ou 8, dans lequel au moins l'un des capteurs est un capteur in situ non invasif choisi dans le groupe constitué par les capteurs ATR-FTIR, les capteurs de température, le capteur SAXS, les capteurs de conductivité, les capteurs de pH et les capteurs de pression.

10. Méthode selon l'une quelconque des revendications 1 à 9, dans lequel l'unité de fonctionnement comprend une unité de chromatographie et/ou une unité de filtration.

11. Méthode selon la revendication 10, dans lequel l'unité de chromatographie est choisie dans le groupe constitué par la chromatographie d'échange d'ions, la chromatographie d'affinité, la chromatographie d'exclusion stérique, la chromatographie en phase inverse, la chromatographie d'interaction hydrophobe, la chromatographie multimodale sur résine, fonctionnant en élution isocratique, linéaire, segmentée et/ou à gradient d'échelon en mode de liaison/éluation ou d'écoulement et l'unité de filtration est choisie dans le groupe constitué de Ultrafiltration, Microfiltration, Nanofiltration, Filtration en profondeur, Fonctionnement en Filtration à flux tangentiel, Filtration sans issue, Filtration à travers des membranes à pores absolus.

12. Méthode selon l'une quelconque des revendications 5 à 11, dans lequel, à l'étape f), la méthode est réglé en ce qui concerne l'une ou l'autre ou une combinaison de la collecte de pointe, de la collecte correcte du produit biologique après repliement, de la filtration ou de la précipitation, de la qualité du produit et de l'entretien de l'équipement de traitement.

13. Méthode selon l'une quelconque des revendications 1 à 12, dans lequel ledit produit biologique est un produit biopharmaceutique, une molécule d'acide nucléique ou une protéine hétérologue, choisie de préférence parmi des protéines thérapeutiques, des enzymes et des peptides, des antibiotiques protéiques, des protéines de fusion, des conjugués glucides-protéines, des protéines structurelles, des protéines régulatrices, des vaccins et des protéines ou particules de type vaccinal, des enzymes de procédé, des facteurs de croissance, des hormones et des cytokines, anticorps ou un métabolite dudit produit biologique.

14. Méthode de production d'un produit biologique comprenant les étapes suivantes :
a. la culture d'un organisme ou d'une cellule capable de produire le produit biologique ;
b. purification du produit résultant de l'étape A), dans laquelle la purification est surveillée selon l'une quelconque des revendications 1 à 13.

15. Dispositif comprenant une unité d'opération pour la purification et/ou la concentration d'un produit biologique, dans lequel l'unité d'opération comprend
une unité de chromatographie et/ou de filtration, dans lequel l'unité de chromatographie ou de filtration comprend au moins un capteur en ligne, et
dans lequel le capteur est connecté à un ordinateur, où les valeurs de données collectées par ledit capteur sont importées dans une base de données et où elles sont évaluées par un modèle d'analyse statistique prédictif multivarié obtenu précédemment à partir de données en ligne obtenues à partir dudit capteur en ligne, et à partir de données hors lignequi ont été obtenus sur la base d'échantillons retirés du processus, effectuant ainsi une analyse statistique multivariée pour la prédiction de la concentration, de la pureté et/ou de la puissance dudit produit biologique et où les valeurs de données réelles sont ensuite diagnostiquées correspondant à la sortie respective de ladite analyse statistique multivariée.

16. Dispositif selon la revendication 15, dans lequel le ou les capteurs en ligne sont choisis dans le groupe constitué de capteurs de diffusion de la lumière multi-angles (MALS), de capteurs d'absorption UV-VIS, de capteurs de fluorescence, de capteurs d'absorption infrarouge (IR), de capteurs de spectroscopie infrarouge à transformée de Fourier à réflexion totale atténuée (ATR-FTIR), de capteurs d'indice de réfraction de la lumière (IR), de capteurs de pH, de capteurs de température, de capteurs de conductivité, de capteurs de pression, de petits angles capteurs de diffusion des rayons X (SAXS) et capteurs redox.
